# EUROPÄISCHE PATENTSCHRIFT

(11) **EP 1 900 792 B1**
(45) Veröffentlichungstag und Bekanntmachung des Hinweises auf die Patenterteilung: **04.08.2010**
(21) Anmeldenummer: 07016045.2
(22) Anmeldetag: 16.08.2007
(51) Int. Cl.: C09K 19/20, C09K 19/30, C09K 19/34, C09K 19/42

(54) **Fluorphenyl-Verbindungen für flüssigkristalline Mischungen**
Fluorine phenyl compounds for liquid crystalline mixtures
Composés de fluorphényle pour mélanges de cristaux liquides

(30) Priorität: 13.09.2006 DE 102006042859
(43) Veröffentlichungstag der Anmeldung: 19.03.2008
(73) Patentinhaber: Merck Patent GmbH, 64293 Darmstadt (DE)
(72) Erfinder: Lietzau, Lars, Dr., 64295 Darmstadt (DE); Czanta, Markus, Dr., 64289 Darmstadt (DE); Hirschmann, Harald, Dr., 64291 Darmstadt (DE); Wittek, Michael, Dr., 64285 Darmstadt (DE)

(56) Entgegenhaltungen:
- EP-A- 0 786 445
- WO-A-01/64667
- WO-A-2007/118623
- DE-A1- 10 229 476
- DE-A1- 10 243 776
- DE-A1-102004 056 901
- US-A- 5 589 102

## Beschreibung

Die Erfindung betrifft Verbindungen der Formel I wie unten definiert, ein Verfahren zu ihrer Herstellung, sowie ihre Verwendung als Komponente(n) in flüssigkristallinen Medien. Darüber hinaus betrifft die vorliegende Erfindung Flüssigkristall- und elektrooptische Anzeigeelemente, welche die erfindungsgemäßen, flüssigkristallinen Medien enthalten. Die erfindungsgemäßen Verbindungen weisen als Strukturelemente eine Difluormethylenoxy-Gruppe an einer in bestimmter Weise fluorierten Biphenylgruppe auf.

In den vergangenen Jahren wurden die Anwendungsgebiete für flüssigkristalline Verbindungen auf verschiedene Arten von Anzeigevorrichtungen, elektrooptische Geräte, elektronische Komponenten, Sensoren, etc. erheblich ausgeweitet. Aus diesem Grund wurden eine Reihe verschiedener Strukturen vorgeschlagen, insbesondere auf dem Gebiet der nematischen Flüssigkristalle. Die nematischen Flüssigkristallmischungen haben bisher die breiteste Anwendung in flachen Anzeigevorrichtungen gefunden. Sie wurden besonders in passiven TN- oder STN-Matrixanzeigen oder Systemen mit einer TFT-Aktivmatrix eingesetzt.

Die erfindungsgemäßen, flüssigkristallinen Verbindungen können als Komponente(n) flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (electrically controlled birefringence), dem IPS-Effekt (in-plane switching) oder dem Effekt der dynamischen Streuung beruhen.

Die Verwendung bestimmter Derivate mit einer Difluormethylenoxy-Brücke (-CF₂O-) als flüssigkristalline Substanzen ist dem Fachmann bekannt. Es wurden bereits verschiedene Verbindungen mit einer Difluormethylenoxy-Brücke als flüssigkristallines Material und dessen Herstellung beschrieben, wie z. B. in der Druckschrift EP 0786445 A1.

In der Patentschrift US 5,589,102 werden Verbindungen mit einer Gruppe -Phenyl-CF₂O- und einer polaren Kopfgruppe als Flüssigkristallkomponente offenbart. Aus der Druckschrift DE 10243776 A1 sind Alkenylverbindungen als Flüssigkristallkomponente bekannt, die eine Gruppe -CF₂O- und optional fluorierte Benzolringe beinhalten können. Aus der Druckschrift DE 10229476 A1 gehen Isothiocyanate hervor, die ebenfalls eine Gruppe -CF₂O- und optional fluorierte Benzolringe beinhalten können. Die Druckschrift DE 102004056901 A1 offenbart flüssigkristalline Mischungen enthaltend Pyranverbindungen, deren allgemeine Formel auch eine Gruppe -CF₂O- und optional fluorierte Benzolringe umfasst.

Der vorliegenden Erfindung lag die Aufgabe zugrunde, neue stabile Verbindungen aufzufinden, die als Komponente(n) flüssigkristalliner Medien geeignet sind. Insbesondere sollen die Verbindungen gleichzeitig eine vergleichsweise geringe Viskosität, sowie eine dielektrische Anisotropie im positiven Bereich besitzen. Für viele aktuelle Mischungskonzepte im Bereich der Flüssigkristalle ist es vorteilhaft, Verbindungen mit einer hohen dielektrischen Anisotropie Δε zu verwenden.

Im Hinblick auf die verschiedensten Einsatzbereiche derartiger Verbindungen mit hohem Δε war es wünschenswert, weitere Verbindungen, vorzugsweise mit hoher Nematogenität zur Verfügung zu haben, die auf die jeweiligen Anwendungen genau maßgeschneiderte Eigenschaften aufweisen.

Der Erfindung lag somit als eine Aufgabe zugrunde, neue stabile Verbindungen aufzufinden, die als Komponente(n) flüssigkristalliner Medien, insbesondere für z. B. TN-, STN-, IPS- und TN-TFT-Displays, geeignet sind.

Eine weitere Aufgabe der vorliegenden Erfindung war es, Verbindungen bereitzustellen, die für sich oder in Mischungen eine hohe dielektrische Anisotropie Δε, einen hohen Klärpunkt sowie eine niedrige Rotationsviskosität γ₁ aufweisen. Darüber hinaus sollten die erfindungsgemäßen Verbindungen unter den in den Anwendungsgebieten vorherrschenden Bedingungen thermisch und photochemisch stabil sein. Ferner sollten die erfindungsgemäßen Verbindungen möglichst eine breite nematische Phase aufweisen. Als Mesogene sollten sie eine breite nematische Phase in Mischungen mit flüssigkristallinen Cokomponenten ermöglichen sowie hervorragend mit nematischen Basismischungen, insbesondere bei tiefen Temperaturen, mischbar sein. Ebenso bevorzugt sind Substanzen mit einem niedrigen Schmelzpunkt und einer geringen Schmelzenthalpie, da diese Größen wiederum Anzeichen für die oben genannten wünschenswerten Eigenschaften sind, wie z. B. eine hohe Löslichkeit, eine breite flüssigkristalline Phase und eine geringe Neigung zur spontanen Kristallisation in Mischungen bei tiefen Temperaturen. Gerade die Löslichkeit bei tiefer Temperatur unter Vermeidung von jeglicher Kristallisation ist wichtig für den sicheren Betrieb und Transport von Anzeigen in Fahr- und Flugzeugen und im Freien.

Überraschenderweise wurde gefunden, dass die erfindungsgemäßen Verbindungen vorzüglich als Komponenten flüssigkristalliner Medien geeignet sind. Mit ihrer Hilfe lassen sich flüssigkristalline Medien für Displays erhalten, die besonders hohe dielektrische Anisotropien benötigen, insbesondere für TN-TFT- und STN-Displays, aber auch für IPS-Systeme oder neuere Konzepte. Die erfindungsgemäßen Verbindungen sind hinreichend stabil und farblos. Auch zeichnen sie sich durch stark positive dielektrische Anisotropien Δε aus, aufgrund derer in der Anwendung in optischen Schaltelementen niedrigere Schwellenspannungen erforderlich sind. Sie besitzen einen besonders breiten nematischen Phasenbereich. Darüber hinaus weisen die erfindungsgemäßen Verbindungen einen hohen Klärpunkt sowie gleichzeitig niedere Werte für die Rotationsviskosität auf. Im Vergleich mit Stoffen aus dem Stand der Technik werden deutlich niedrigere Schmelzpunkte und Schmelzenthalpien beobachtet.

Mit der Bereitstellung der erfindungsgemäßen Verbindungen wird ganz allgemein die Palette der flüssigkristallinen Substanzen, die sich unter verschiedenen, anwendungstechnischen Gesichtspunkten zur Herstellung flüssigkristalliner Mischungen eignen, erheblich verbreitert.

Die erfindungsgemäßen Verbindungen besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind. Es können aber auch den erfindungsgemäßen Verbindungen flüssigkristalline Basismaterialien aus anderen Verbindungsklassen zugesetzt werden, um beispielsweise die dielektrische und/oder die optische Anisotropie eines solchen Dielektrikums zu beeinflussen und/oder um dessen Schwellenspannung und/oder dessen Viskosität zu optimieren.

Gegenstand der Erfindung sind somit Verbindungen der Formel I, 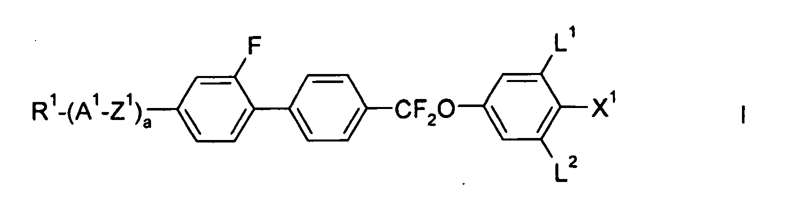 worin
- R¹: H, F, Cl, Br, einen halogenierten oder unsubstituierten Alkylrest mit 1 bis 15 C-Atomen, wobei in diesen Resten auch eine oder mehrere CH₂-Gruppen jeweils unabhängig voneinander durch -C≡C-, -(CO)O-, -O(CO)-, -(CO)- oder -0- so ersetzt sein können, dass O-Atome nicht direkt miteinander verknüpft sind,
- A¹: a) trans-1,4-Cyclohexylen oder Cyclohexenylen, worin auch eine oder mehrere nicht benachbarte CH₂-Gruppen durch -O- und/oder -S- ersetzt sein können und worin H durch F substituiert sein kann,
b) 1,4-Phenylen, worin eine oder zwei CH-Gruppen durch N ersetzt sein können und worin auch ein oder mehrere H- Atome gegen Br, Cl, F, CN, Methyl, Methoxy oder eine ein- oder mehrfach fluorierte Methyl- oder Methoxygruppe ersetzt sein können,
- Z¹: eine Einfachbindung, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- oder -C≡C-, wobei asymmetrische Brücken nach beiden Seiten orientiert sein können,
- L¹ und L²: unabhängig voneinander H oder F,
- X¹: F, Cl, CN, SF₅, einen halogenierten Alkylrest, halogenierten Alkoxyrest, halogenierten Alkenylrest oder halogenierten Alkenyloxyrest mit jeweils bis zu 7 C-Atomen, und
- a: 0, 1 oder 2, bevorzugt 0 oder 1,
bedeuten.

Gegenstand der Erfindung ist weiterhin die Verwendung der Verbindungen der Formel I in flüssigkristallinen Medien.

Ebenfalls Gegenstand der vorliegenden Erfindung sind flüssigkristalline Medien mit mindestens zwei flüssigkristallinen Komponenten, welche mindestens eine Verbindung der Formel I enthalten.

Die Verbindungen der Formel I sind in reinem Zustand farblos und bilden für sich oder in Mischungen flüssigkristalline Mesophasen in einem für die elektrooptische Verwendung günstig gelegenen Temperaturbereich. Mit den erfindungsgemäßen Verbindungen lassen sich breite nematischen Phasenbereiche erzielen. In flüssigkristallinen Mischungen unterdrücken die erfindungsgemäßen Substanzen die smektischen Phasen und führen zu einer deutlichen Verbesserung der Tieftemperatur-Lagerstabilität.

Bevorzugt sind Verbindungen der Formel I, worin a 0 oder 1, insbesondere a = 0, ist.

Z¹ bedeutet, soweit vorhanden, bevorzugt eine Einfachbindung, -CF₂O-, -OCF₂-, -C₂F₄-, -CH₂O-, -OCH₂- oder -(CO)O-, insbesondere eine Einfachbindung.

A¹ bedeutet bevorzugt 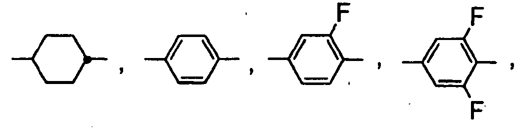 und ferner,

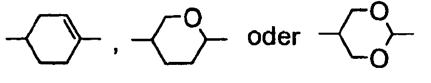

Die Strukturelemente A¹ und Z¹ können für den Fall, dass sie mehrfach auftreten (a > 1), jeweils die gleiche oder unterschiedliche Bedeutungen annehmen.

R¹ bedeutet bevorzugt Alkyl oder Alkoxy mit bis zu 8 Kohlenstoffatomen.
R¹ bedeutet besonders bevorzugt unverzweigtes Alkyl.

X¹ bedeutet bevorzugt F, Cl, CN, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₃, OCFHCF₂CF₃, OCFHCF₂CHF₂, OCF₂CF₂CF₃, OCF₂CF₂CClF₂, OCClFCF₂CF₃ oder CH=CHF₂. Ganz besonders ist es bevorzugt, dass X¹ in den erfindungsgemäßen Verbindungen der Formel I eine Gruppe aus F, Cl, CF₃, OCF₃, OCHF₂ oder CN bedeutet, darunter insbesondere F oder OCF₃.

Besonders bevorzugt sind Verbindungen der Formeln IA 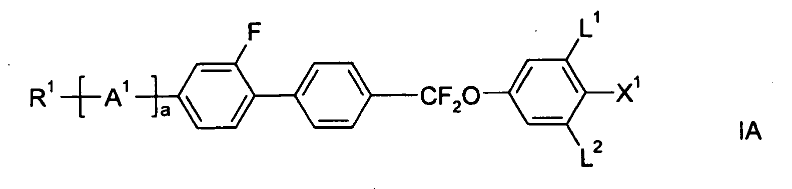 worin
R¹, A¹, L¹, L², X¹ und a die oben für Formel I angegebenen Bedeutungen haben.

Bevorzugt sind Verbindungen der Formeln I und IA, worin wenigstens eines aus L¹ und L² ein Fluor bedeutet, insbesondere solche, worin beide F bedeuten.

Besonders bevorzugte Verbindungen der Formel I bzw. IA sind die Verbindungen der Formeln I1 bis I8, 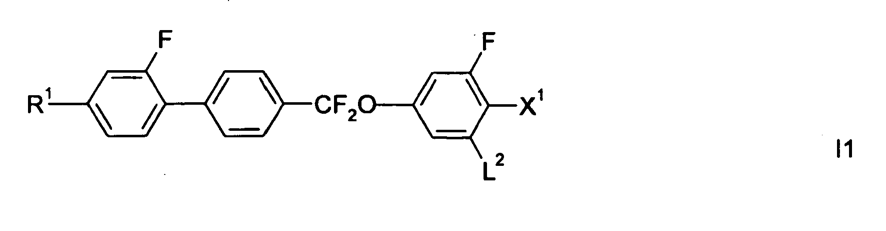 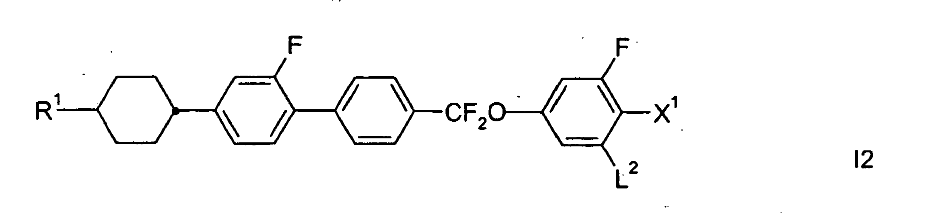 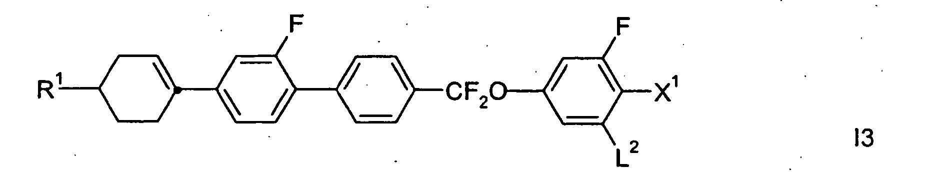 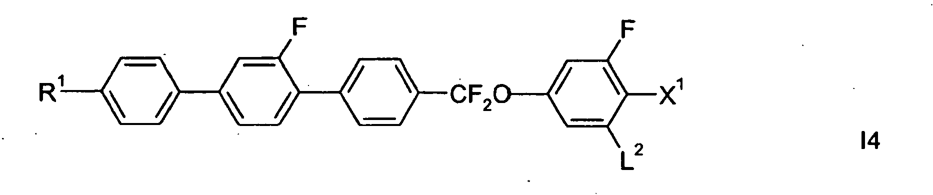 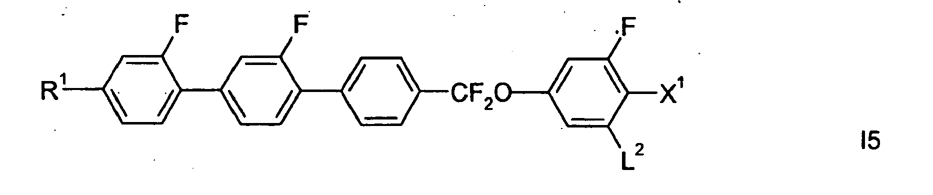 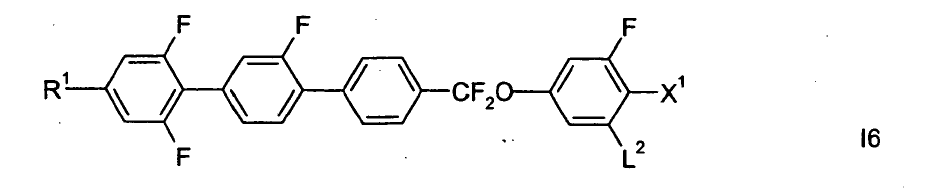 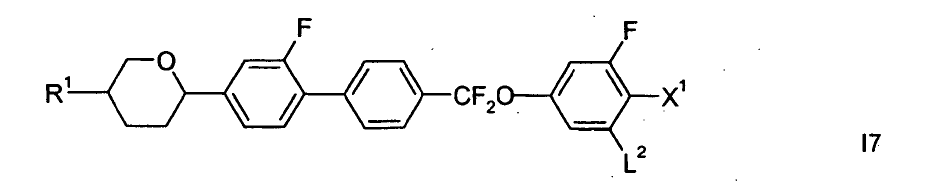 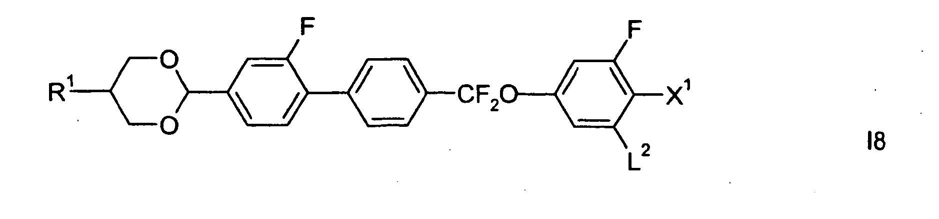 worin R¹ und X¹ die oben angegebenen Bedeutungen, insbesondere die bevorzugten Bedeutungen oder Kombinationen davon, haben. L² bedeutet bevorzugt F. Unter den Verbindungen der Formeln 11 bis 18 sind die Formeln I1, I2 und 14, ferner I7 bevorzugt, insbesondere die Verbindungen der Formel I1 und I4.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel I können vorteilhafterweise wie an den folgenden beispielhaften Synthesen und den Beispielen ersichtlich hergestellt werden (Schema 1):

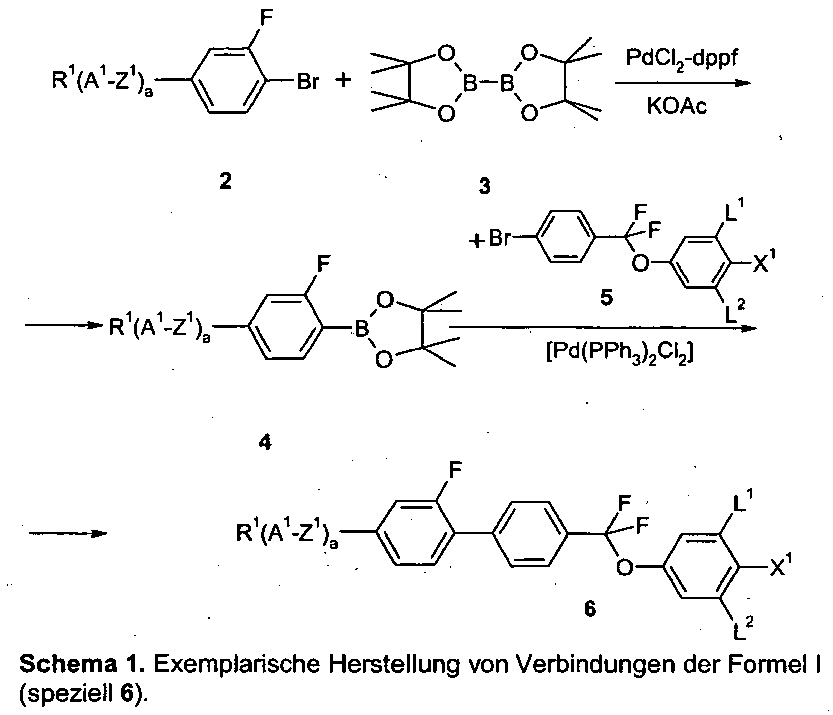

Entsprechende Ausgangsprodukte lassen sich in der Regel vom Fachmann ohne weiteres über literaturbekannte Synthesemethoden herstellen. Die Alkylreste des Boronsäureesters **4** können durch andere Alkylgruppen etc. oder durch Wasserstoffatome ersetzt werden. Die Synthese der entsprechenden Boronsäuren oder Boronsäureester analog zu **4** erfolgt in analoger Weise oder durch Metallierung von 2 bei sehr tiefen Temperaturen und anschließender Reaktion mit geeigneten einfachen Borverbindungen, wie z. B. Borsäurealkylestern.

Der Synthesebaustein **5** wird nach dem in der Druckschrift WO 01/064667 A1 beschrieben Verfahren hergestellt: p-Brombenzoesäure wird am Wasserabscheider in Gegenwart von Propandithiol und Trifluormethansulfonsäure in das entsprechende Dithianyliumtriflat übergeführt. Das Triflat wird in einer oxidativen Fluorierung mit DBH und Triethylamin-trishydrofluorid als Fluorierungsreagenz zum Synthesebaustein **5** umgesetzt (Schema 2).

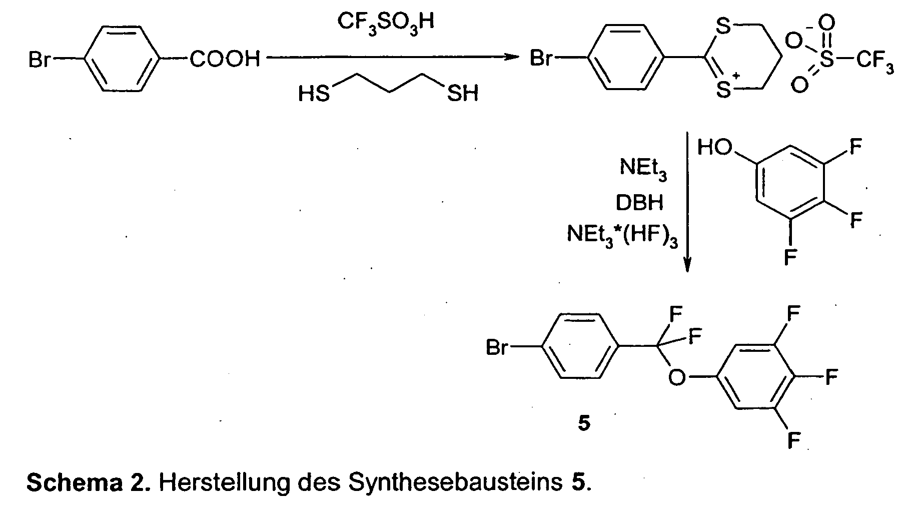

Anstelle der Bromide in Verbindung **5** können auch Iodide oder Abgangsgruppen mit vergleichbarer Reaktivität verwendet werden (z. B. die Triflat-Gruppe).
Die Erfindung hat daher auch ein Verfahren zur Herstellung von Verbindungen der Formel I zum Gegenstand das einen Verfahrensschritt umfasst, in dem eine Boronsäure der Formel IIA oder ein offenkettiger oder cyclischer Boronsäureester der Formel IIB 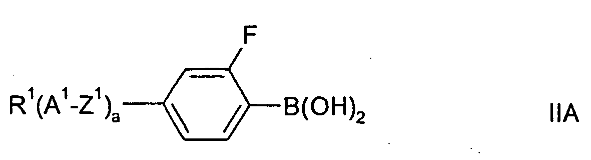 oder 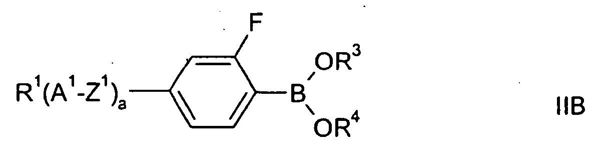 worin
R¹, A¹, Z¹ und a wie für Formel I definiert sind, und
R³, R⁴ ein Alkyl mit 1-12 C-Atomen oder R³+R⁴ zusammen auch ein 2-10 C Alkylen, insbesondere der Formeln

   -CH₂-(CH₂)ₚ-CH₂- und -C(CH₃)₂C(CH₃)₂-,

   oder 1,2-Phenylen bedeuten,
   wobei Phenylen, R³, R⁴ und R³+R⁴ auch substituiert sein können und wobei p 0 oder 1 ist,
mit einer Verbindung der Formel III 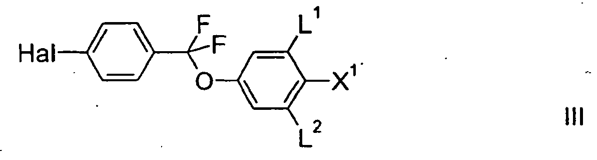 worin
L¹, L² und X¹ wie für Formel I definiert sind und
Hal Cl, Br, I oder O(SO₂)CF₃ bedeutet,
in Gegenwart eines Übergangsmetallkatalysators zur Reaktion gebracht wird. Dabei entsteht eine Verbindung der Formel I.

Der Übergangsmetallkatalysator ist bevorzugt ein Palladiumkomplex der Oxidationsstufen 0, II oder IV. Die Reaktion erfolgt bevorzugt in homogener Phase mit einem löslichen Katalysator. Bei den Komplexen handelt es sich besonders bevorzugt um Bis(triphenylphosphin)-palladium(II)chlorid. Die verwendeten Reaktionsmethoden und Reagenzien sind prinzipiell literaturbekannt. Weitere Reaktionsbedingungen können den Ausführungsbeispielen entnommen werden.

Eine Alternative zu dem dargestellten Verfahren besteht darin, dass die reaktiven Gruppen der Reaktionspartner (Boronsäure-Derivat und Halogenid) ausgetauscht werden.
Daraus ergibt sich ein weiteres erfindungsgemäßes Verfahren zur Herstellung von Verbindungen der Formel I das einen Verfahrensschritt umfasst, in dem eine Verbindung mit einer Abgangsgruppe Hal' der Formel IV 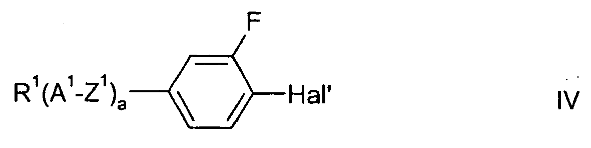 worin
R¹, A¹, Z¹ und a wie für Formel I definiert sind, und
Hal' -O(SO₂)CF₃, Cl, Br oder I bedeutet,
mit einer Boronsäure oder einem offenkettigen oder cyclischen Boronsäureester der Formel V 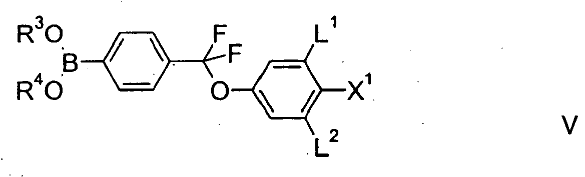 worin
L¹, L² und X¹ wie für Formel I definiert sind und
R³, R⁴ H, ein Alkyl mit 1-12 C-Atomen oder R³+R⁴ zusammen auch ein 2-10 C Alkylen, insbesondere der Formeln

   -CH₂-(CH₂)ₚ-CH₂- und -C(CH₃)₂C(CH₃)₂-,

   oder 1,2-Phenylen bedeuten,
   wobei Phenylen, R³, R⁴ und R³+R⁴ auch substituiert sein können und wobei p 0 oder 1 ist,
in Gegenwart eines Übergangsmetallkatalysators zur Reaktion gebracht wird.

Phenylen, R³, R⁴ oder R³+R⁴ können ein- oder mehrfach durch einfache unreaktive Gruppen wie 1-5 C Alkyl oder Alkoxy, Cl, CN, -(CO)OAlkyl, etc. substituiert sein.

Weitere, vorangehend nicht genannte, bevorzugte Verfahrensvarianten lassen sich den Beispielen oder den Ansprüchen entnehmen.

Gegenstand der Erfindung sind auch flüssigkristalline Medien enthaltend eine oder mehrere der erfindungsgemäßen Verbindungen der Formel I. Die flüssigkristallinen Medien enthalten wenigstens zwei Komponenten.

Man erhält sie vorzugsweise indem man die Komponenten miteinander vermischt. Ein erfindungsgemäßes Verfahren zur Herstellung eines flüssigkristallinen Mediums ist daher dadurch gekennzeichnet, dass man mindestens eine Verbindung der Formel I mit mindestens einer weiteren mesogenen Verbindung vermischt und gegebenenfalls Additive zugibt.

Die erzielbaren Kombinationen aus Klärpunkt, Viskosität bei tiefer Temperatur, thermischer und UV-Stabilität und dielektrischer Anisotropie übertreffen bei weitem bisherige Materialien aus dem Stand der Technik.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexane, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile der erfindungsgemäßen Medien in Frage kommenden Verbindungen lassen sich durch die Formeln 1, 2, 3, 4 und 5 charakterisieren:

R'-L-E-R" 1

R'-L-COO-E-R" 2

R'-L-CF₂O-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus den Strukturelementen -Phe-, -Cyc-, -Phe-Phe-, -Phe-Cyc-, -Cyc-Cyc-, -Pyr-, -Dio-, -Py-, -G-Phe-, -G-Cyc- und deren Spiegelbildern gebildeten Gruppe, wobei Phe unsubstituiertes oder durch Fluor substituiertes 1,4-Phenylen, Cyc trans-1,4-Cyclohexylen, Pyr Pyrimidin-2,5-diyl oder Pyridin-2,5-diyl, Dio 1,3-Dioxan-2,5-diyl, Py Tetrahydropyran-2,5-diyl- und G 2-(trans-1,4-Cyclohexyl)-ethyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe, Py und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, -F, -Cl, -CN, -NCS oder -(O)ᵢCH₃₋ₖFₖ, wobei i 0 oder 1 und k 1, 2 oder 3 ist.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢCH₃₋ₖFₖ, wobei i 0 oder 1 und k 1, 2 oder 3 ist. Die Verbindungen, in denen R" diese Bedeutung hat, werden mit den Teilformeln 1 b, 2b, 3b, 4b und 5b bezeichnet. Besonders bevorzugt sind solche Verbindungen der Teilformeln 1 b, 2b, 3b, 4b und 5b, in denen R" die Bedeutung -F, -Cl, -NCS, -CF₃, -OCHF₂ oder -OCF₃ hat.

In den Verbindungen der Teilformeln 1 b, 2b, 3b, 4b und 5b hat R' die bei den Verbindungen der Teilformeln 1a bis 5a angegebenen Bedeutungen und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN. Diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a bis 5a angegebenen Bedeutungen und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus den Gruppen A, B und/oder C. Die Massenanteile der Verbindungen aus diesen Gruppen in den erfindungsgemäßen Medien sind vorzugsweise:

| | |
|---|---|
| Gruppe A: | 0 bis 90 %, vorzugsweise 20 bis 90 %, besonders bevorzugt 30 bis 90 %; |
| Gruppe B: | 0 bis 80 %, vorzugsweise 10 bis 80 %, besonders bevorzugt 10 bis 65 %; |
| Gruppe C: | 0 bis 80 %, vorzugsweise 0 bis 80 %, besonders bevorzugt 0 bis 50 %; |

wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die erfindungsgemäßen Medien enthalten vorzugsweise 1 bis 40 %, besonders bevorzugt 5 bis 30 %, der erfindungsgemäßen Verbindungen.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe, chirale Dotierstoffe, Stabilisatoren oder Nanopartikel zugesetzt werden. Die einzelnen zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben. Die erfindungsgemäßen Flüssigkristallmischungen ermöglichen eine bedeutende Erweiterung des zur Verfügung stehenden Parameterraumes.

Gegenstand der Erfindung sind auch elektrooptische Anzeigen (insbesondere TFT-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Der Ausdruck "Alkyl" umfasst unverzweigte und verzweigte Alkylgruppen mit 1-9 Kohlenstoffatomen, insbesondere die unverzweigten Gruppen Methyl, Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" umfasst unverzweigte und verzweigte Alkenylgruppen mit bis zu 9 Kohlenstoffatomen, insbesondere die unverzweigten Gruppen. Besonders bevorzugte Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1 E-Alkenyl, C₄-C₇-3E-Alkenyl und C₅-C₇-4-Alkenyl. Beispiele bevorzugter Alkenylgruppen sind Vinyl, 1 E-Propenyl, 1 E-Butenyl, 1 E-Pentenyl, 1 E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "halogenierter Alkylrest" umfasst vorzugsweise ein- oder mehrfach fluorierte und/oder chlorierte Reste. Perhalogenierte Reste sind eingeschlossen. Besonders bevorzugt sind fluorierte Alkylreste, insbesondere CF₃, CH₂CF₃, CH₂CHF₂, CHF₂, CH₂F, CHFCF₃ und CF₂CHFCF₃. Der Ausdruck "halogenierter Alkenylrest" und verwandte Ausdrücke erklären sich entsprechend.

Die Gesamtmenge an Verbindungen der Formeln I in den erfindungsgemäßen Gemischen ist nicht kritisch. Die Gemische können daher eine oder mehrere weitere Komponenten enthalten zwecks Optimierung verschiedener Eigenschaften.

Der Aufbau der erfindungsgemäßen Matrix-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der Matrix-Anzeige, insbesondere auch Matrix-Anzeigeelemente auf Basis von poly-Si TFT.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die folgenden Beispiele erläutern die Erfindung, ohne sie begrenzen zu sollen. Der Fachmann wird in der Lage sein, den Beispielen Details zur Durchführung zu entnehmen, die in der allgemeinen Beschreibung nicht im Einzelnen aufgeführt sind, sie nach allgemeinen Fachkenntnissen zu verallgemeinern und auf eine spezielle Problemstellung anzuwenden.

Vor- und nachstehend bedeuten Prozentangaben Gewichtsprozent. Alle Temperaturen sind in Grad Celsius angegeben. Ferner bedeuten K = kristalliner Zustand, N = nematische Phase, Sm = smektische Phase und I = isotrope Phase. Die Angaben zwischen diesen Symbolen stellen die Übergangstemperaturen dar. Δn bedeutet optische Anisotropie (589 nm, 20 °C), Δε die dielektrische Anisotropie (1 kHz, 20 °C) und γ₁ die Rotationsviskosität (20°C; in der Einheit mPa·s).

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.
Die dielektrische Anisotropie Δε der einzelnen Substanzen wird bei 20 °C und 1 kHz bestimmt. Dazu werden 5-10 Gew.% der zu untersuchenden Substanz in der dielektrisch positiven Mischung ZLI-4792 (Merck KGaA) gelöst gemessen und der Messwert auf eine Konzentration von 100 % extrapoliert. Die optische Anisotropie Δn wird bei 20°C und einer Wellenlänge von 589,3 nm bestimmt, die Rotationsviskosität γ₁ bei 20°C, beide ebenfalls durch lineare Extrapolation.

Folgende Abkürzungen werden verwendet:

| | |
|---|---|
| p-TsOH | *p*-Toluolsulfonsäure |
| THF | Tetrahydrofuran |
| MTB-Ether | Methyl-*t*-butylether |
| DBH | 1,3-Dibrom-5,5-dimethylhydanthoin |
| Cyc | Cyclohexyl |
| dppf | 1,1'-Bis(diphenylphosphanyl)ferrocen) |
| DMAP | 4-(*N,N*-Dimethylamino)-pyridin |
| RT | Raumtemperatur |

### Beispiel 1

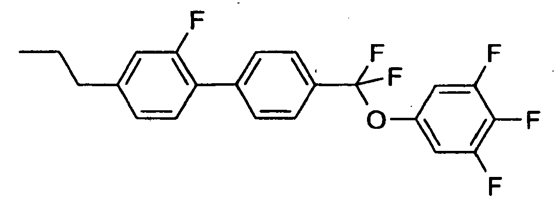

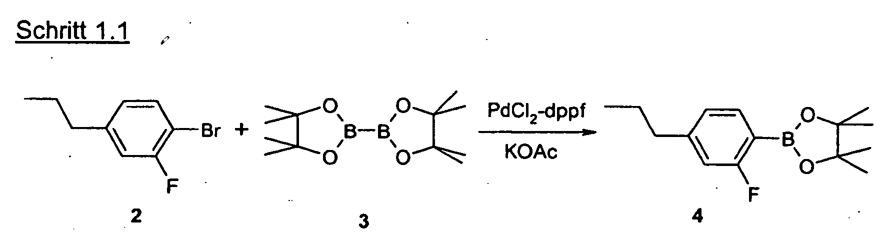

Eine Lösung von 50 g (230 mmol) des Bromids **2** in 590 ml Dioxan wird mit 71 g (276 mmol) der Borverbindung **3**, 67 g (690 mmol) Kaliumacetat und 5 g (7 mmol) PdCl₂-dppf versetzt und 17 h auf 100 °C erwärmt. Der abgekühlte Ansatz wird mit Wasser versetzt und mit MTB-Ether extrahiert. Die organische Phase wird über Natriumsulfat getrocknet und eingeengt. Der Rückstand wird über Kieselgel (n-Heptan) filtriert. Der erhaltene Rückstand **4** wird ohne weitere Reinigung in der Folgestufe eingesetzt.

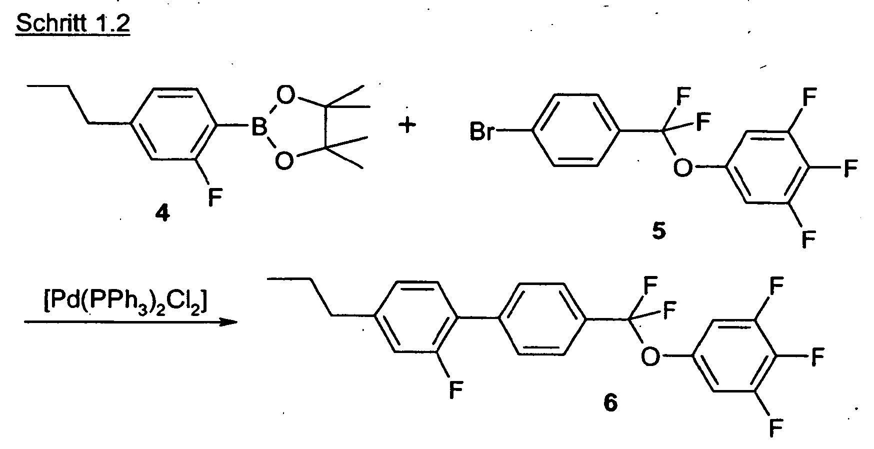

21 g (75 mmol) Natriummetaborat-Octahydrat werden in 38 ml Wasser vorgelegt und mit 40 ml THF, 0,15 ml (1mmol) Hydraziniumhydroxid und 0,7 g (1 mmol) Bis(triphenylphosphin)-palladium(II)chlorid versetzt und 5 min bei RT gerührt. Anschließend wird eine Lösung von 14,8 g (89%ig; 50 mmol) des Boronsäureesters **4** und 17,7 g (50 mmol) des Bromids **5** in den Ansatz gegeben. Nach **6** h Rühren unter Rückfluss wird das Reaktionsgemisch mit MTB-Ether verdünnt. Die organische Phase wird eingeengt. Der Rückstand wird über Kieselgel (n-Heptan) filtriert. Die Endreinigung des Produktes erfolgt durch Kristallisation aus Heptan.
K 26 I
Δε 20
Δn 0,131
γ₁ 62 mPa·s

Analog werden die folgenden Verbindungen der Formel hergestellt (Tabelle 1):

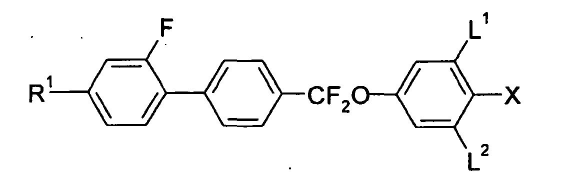

**Tabelle 1**

| R¹ | X | L¹ | L² | Werte |
|---|---|---|---|---|
| H | F | F | H | |
| CH₃ | F | F | H | |
| C₂H₅ | F | F | H | |
| n-C₃H₇ | F | F | H | K 25 N (14) I, Δε 14, |
| | | | | Δn 0,143, γ₁ 119 mPa·s |
| n-C₃H₇ | F | H | H | K 40 N 57 I, Δε 10, |
| | | | | Δn 0,156, γ₁ 121 mPa·s |
| n-C₄H₉ | F | F | H | |
| n-C₅H₁₁ | F | F | H | |
| n-C₆H₁₃ | F | F | H | |
| n-C₇H₁₅ | F | F | H | |
| H | F | F | F | |
| CH₃ | F | F | F | |
| C₂H₅ | F | F | F | K 49 I, Δε 21, Δn 0,124, |
| | | | | γ₁ 51 mPa·s |
| n-C₃H₇ | F | F | F | vgl. Beispiel 1 |
| n-C₄H₉ | F | F | F | K 22 I, Δε 18, Δn 0,128, |
| | | | | γ1 102 mPa·s |
| n-C₅H₁₁ | F | F | F | K 14 N (-5) I, Δε 18, |
| | | | | Δn 0,124, γ1 121 mPa·s |
| n-C₆H₁₃ | F | F | F | |
| n-C₇H₁₅ | F | F | F | |
| H | OCF₃ | F | H | |
| CH₃ | OCF₃ | F | H | |
| C₂H₅ | OCF₃ | F | H | |
| n-C₃H₇ | OCF₃ | F | H | K 41 SmA (38) N (40) I, |
| | | | | Δε 17, An 0,142, |
| | | | | γ₁ 131 mPa·s |
| n-C₄H₉ | OCF₃ | F | H | |
| n-C₅H₁₁ | OCF₃ | F | H | |
| n-C₆H₁₃ | OCF₃ | F | H | |
| n-C₇H₁₅ | OCF₃ | F | H | |
| H | OCF₃ | F | F | |
| CH₃ - | OCF₃ | F | F | |
| C₂H₅ | OCF₃ | F | F | |
| n-C₃H₇ | OCF₃ | F | F | K 41 SmA (21) I, Δε 21, |
| | | | | Δn 0, 134, γ₁ 147 mPa·s |
| n-C₄H₉ | OCF₃ | F | F | |
| n-C₅H₁₁ | OCF₃ | F | F | |
| n-C₆H₁₃ | OCF₃ | F | F | |
| n-C₇H₁₅ | OCF₃ | F | F | |
| H | Cl | F | H | |
| CH₃ | Cl | F | H | |
| C₂H₅ | Cl | F | H | |
| n-C₃H₇ | Cl | F | H | |
| n-C₄H₉ | Cl | F | H | |
| n-C₅H₁₁ | Cl | F | H | |
| n-C₆H₁₃ | Cl | F | H | |
| n-C₇H₁₅ | Cl | F | H | |
| H | Cl | F | F | |
| CH₃ | Cl | F | F | |
| C₂H₅ | Cl | F | F | |
| n-C₃H₇ | Cl | F | F | |
| n-C₄H₉ | Cl | F | F | |
| n-C₅H₁₁ | Cl | F | F | |
| n-C₆H₁₃ | Cl | F | F | |
| n-C₇H₁₅ | Cl | F | F | |
| H | CN | F | H | |
| CH₃ | CN | F | H | |
| C₂H₅ | CN | F | H | |
| n-C₃H₇ | CN | F | H | |
| n-C₄H₉ | CN | F | H | |
| n-C₅H₁₁ | CN | F | H | |
| n-C₆H₁₃ | CN | F | H | |
| n-C₇H₁₅ | CN | F | H | |
| H | CN | F | F | |
| CH₃ | CN | F | F | |
| C₂H₅ | CN | F | F | |
| n-C₃H₇ | CN | F | F | |
| n-C₄H₉ | CN | F | F | |
| n-C₅H₁₁ | CN | F | F | |
| n-C₆H₁₃ | CN | F | F | |
| n-C₇H₁₅ | CN | F | F | |
| H | OCHF₂ | F | H | |
| CH₃ | OCHF₂ | F | H | |
| C₂H₅ | OCHF₂ | F | H | |
| n-C₃H₇ | OCHF₂ | F | H | |
| n-C₄H₉ | OCHF₂ | F | H | |
| n-C₅H₁₁ | OCHF₂ | F | H | |
| n-C₆H₁₃ | OCHF₂ | F | H | |
| n-C₇H₁₅ | OCHF₂ | F | H | |
| H | OCHF₂ | F | F | |
| CH₃ | OCHF₂ | F | F | |
| C₂H₅ | OCHF₂ | F | F | |
| n-C₃H₇ | OCHF₂ | F | F | |
| n-C₄H₉ | OCHF₂ | F | F | |
| n-C₅H₁₁ | OCHF₂ | F | F | |
| n-C₆H₁₃ | OCHF₂ | F | F | |
| n-C₇H₁₅ | OCHF₂ | F | F | |
| H | CF₃ | F | H | |
| CH₃ | CF₃ | F | H | |
| C₂H₅ | CF₃ | F | H | |
| n-C₃H₇ | CF₃ | F | H | |
| n-C₄H₉ | CF₃ | F | H | |
| n-C₅H₁₁ | CF₃ | F | H | |
| n-C₆H₁₃ | CF₃ | F | H | |
| n-C₇H₁₅ | CF₃ | F | H | |
| H | CF₃ | F | F | |
| CH₃ | CF₃ | F | F | |
| C₂H₅ | CF₃ | F | F | |
| n-C₃H₇ | CF₃ | F | F | K 44 I, Δε 27, Δn 0,132, |
| | | | | γ₁ 180 mPa·s |
| n-C₄H₉ | CF₃ | F | F | |
| n-C₅H₁₁ | CF₃ | F | F | |
| n-C₆H₁₃ | CF₃ | F | F | |
| n-C₇H₁₅ | CF₃ | F | F | |

### Beispiel 2

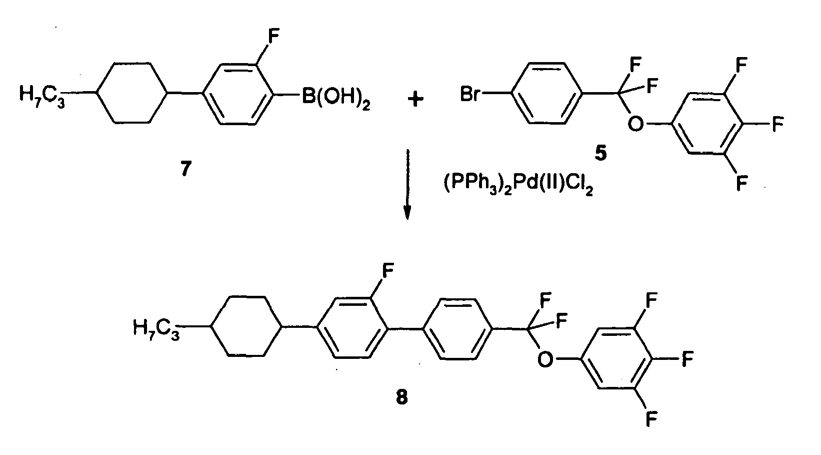

14,6 g (53 mmol) Natriummetaborat-Octahydrat werden in 52 ml Wasser und 50 ml THF vorgelegt und mit 1,0 g (1,4 mmol) (PPh₃)₂Pd(II)Cl₂ und 0,07 ml (1,4 mmol) Hydraziniumhydroxid versetzt. Nach 5 min werden 18,5 g (70 mmol) der Boronsäure **7**, 24,7 g (70 mmol) des Bromids **5** und 56 ml THF in den Ansatz gegeben, der 6 h zum Sieden erhitzt wird. Die abgekühlte Reaktionslösung wird mit 200 ml MTB-Ether verdünnt. Die organische Phase wird angetrennt und eingeengt. Der Rückstand wird über Kieselgel (Toluol/n-Heptan 1:1) gegeben. Die Endreinigung erfolgt durch Kristallisation aus n-Heptan.
K 76 N 161 I
Δε 19
Δn 0,158

### Beispiel 3

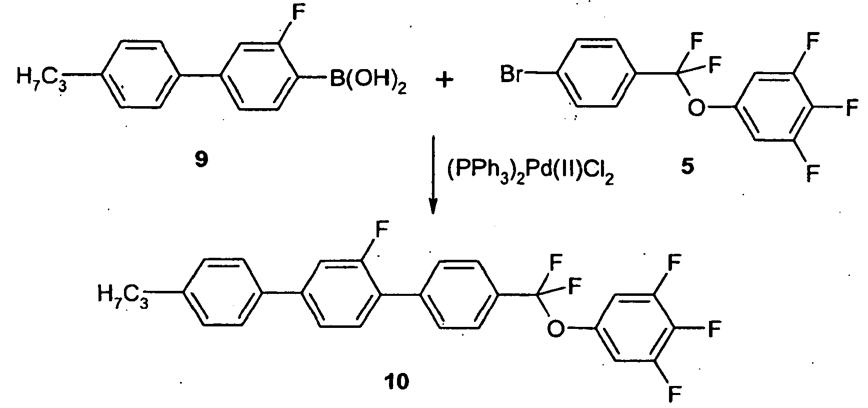

13,9 g (50 mmol) Natriummetaborat-Octahydrat werden in 37 ml Wasser und 40 ml THF vorgelegt und mit 716 mg (1 mmol) (PPh₃)₂Pd(II)Cl₂ und 0,05 ml (1 mmol) Hydraziniumhydroxid versetzt. Nach 5 min werden 12,9 g (50 mmol) der Boronsäure **9**, 17,7 g (50 mmol) des Bromids **5** und 90 ml THF in den Ansatz gegeben, der 6 h zum Sieden erhitzt wird. Die abgekühlte Reaktionslösung wird mit 200 ml MTB-Ether verdünnt. Die organische Phase wird angetrennt und eingeengt. Der Rückstand wird über Kieselgel (Toluol/*n*-Heptan 1:1) gegeben. Die Endreinigung erfolgt durch Kristallisation aus n-Heptan/MTB-Ether 4:1.
K 102 SmA 150 N 178 l
Δε 22
Δ n 0,240

### Beispiel 4

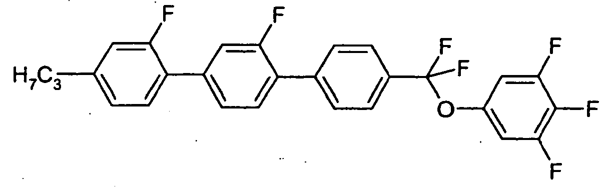

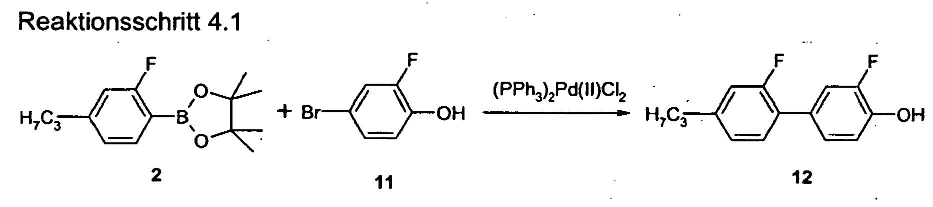

19,0 g (69 mmol) Natriummetaborat-Octahydrat werden in 27 ml Wasser und 40 ml THF vorgelegt und mit 643 mg (0,9 mmol) (PPh₃)₂Pd(II)Cl₂ und 0,05 ml (1 mmol) Hydraziniumhydroxid versetzt. Nach 5 min werden 10,9 g (37 mmol) des Boronesters **2**, 7,5 g (38 mmol) des Phenols **11** und 65 ml THF in den Ansatz gegeben, der 8 h zum Sieden erhitzt wird. Die abgekühlte Reaktionslösung wird mit 100 ml MTB-Ether verdünnt. Die organische Phase wird angetrennt und eingeengt. Der Rückstand wird ohne weitere Reinigung eingesetzt.

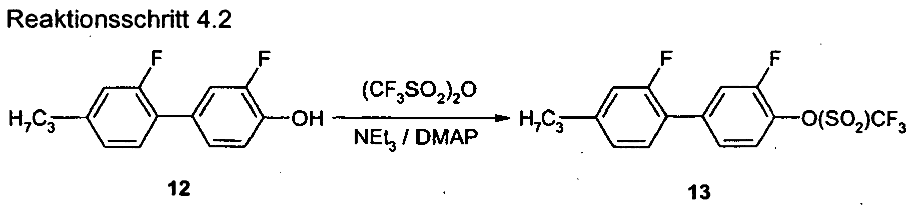

Bei 0°C wird eine Lösung von 14,4 g (42 mmol) des Phenols **12,** 10 ml Triethylamin und 125 mg DMAP in 150 ml Dichlormethan mit 8,3 ml (51 mmol) Trifluormethansulfonsäureanhydrid versetzt und anschließend 18 h bei RT gerührt. Der Ansatz wird zweimal mit Eiswasser gewaschen und eingeengt. Der Rückstand wird über Kieselgel (Toluol/*n*-Heptan 1:4) gegeben. Die erhaltene Flüssigkeit wird ohne weitere Reinigung umgesetzt.

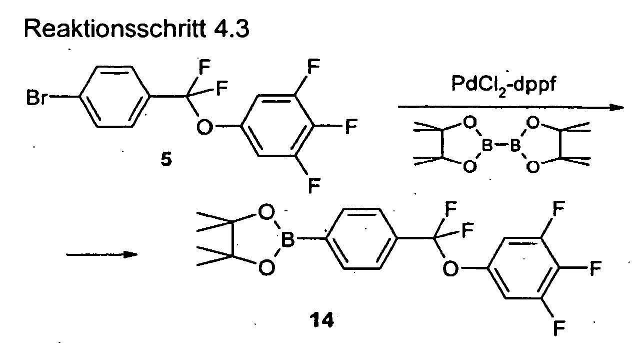

Eine Lösung von 122 g (350 mmol) des Bromids **5** in 900 ml Dioxan wird mit 135 g (520 mmol) der Borverbindung **3**, 102 g (1,04 mol) Kaliumacetat und 7,6 g (10 mmol) PdCl₂-dppf versetzt und 4 h auf 100 °C erwärmt. Der abgekühlte Ansatz wird mit Wasser versetzt und MTB-Ether extrahiert. Die organische Phase wird über Natriumsulfat getrocknet und eingeengt. Der Rückstand wird über Kieselgel (Toluol) filtriert und aus Ethanol kristallisiert.

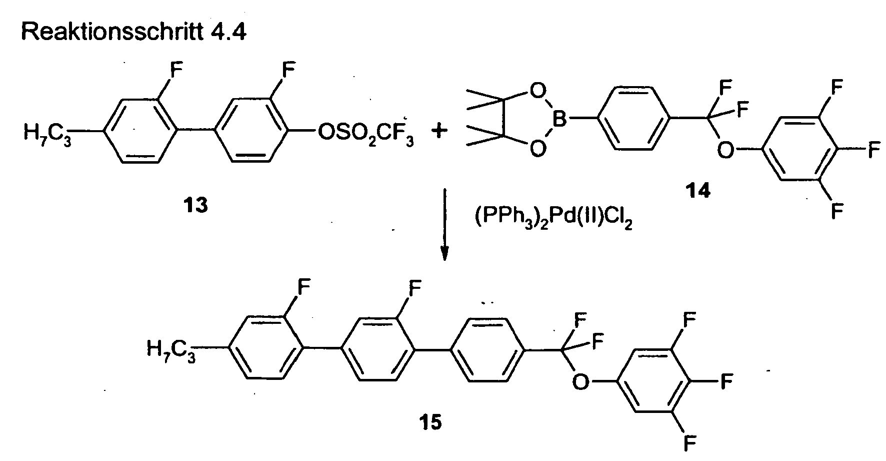

10,7 g (37 mmol) Natriummetaborat-Octahydrat werden in 15 ml Wasser und 15 ml THF vorgelegt und mit 351 mg (0,5 mmol) (PPh₃)₂Pd(II)Cl₂ und 0,03 ml (0,5 mmol) Hydraziniumhydroxid versetzt. Nach 5 min werden 10,0 g (24 mmol) des Boronesters **14**, 9,2 g (24 mmol) des Triflats 13 und 90 ml THF in den Ansatz gegeben, der 6 h zum Sieden erhitzt wird. Die abgekühlte Reaktionslösung wird mit 100 ml MTB-Ether verdünnt. Die organische Phase wird angetrennt und eingeengt. Der Rückstand wird über Kieselgel (Toluol) gegeben. Die Endreinigung erfolgt durch Kristallisation aus n-Heptan.
K 93 SmA 104 N 152 l
Δε 24
Δ n 0,220

### Beispiel 5

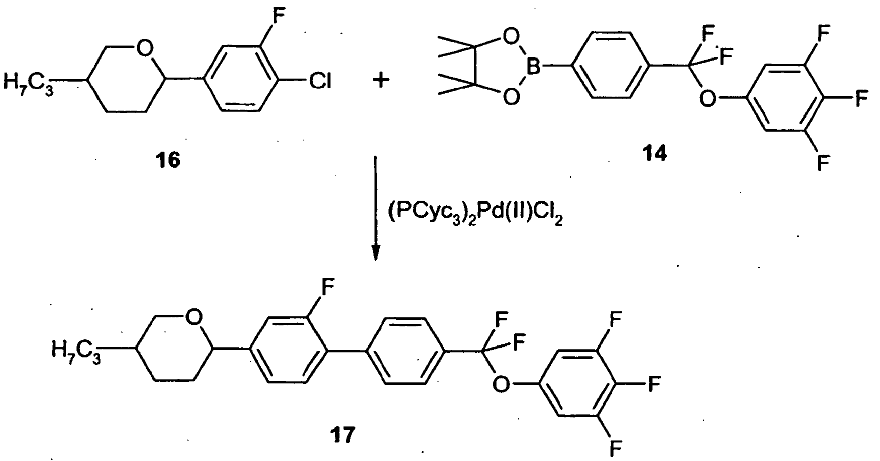

13,9 g (50 mmol) Natriummetaborat-Octahydrat werden in 19 ml Wasser und 18 ml THF vorgelegt und mit 738 mg (1 mmol) (PCyc₃)₂Pd(II)Cl₂ und 0,1 ml (1 mmol) Hydraziniumhydroxid versetzt. Nach 5 min werden 20,8 g (80 mmol) des Boronesters **14**, 13,7 g (50 mmol) des Chlorids **16** und 55 ml THF in den Ansatz gegeben, der 8 h zum Sieden erhitzt wird. Die abgekühlte Reaktionslösung wird mit 200 ml MTB-Ether verdünnt. Die organische Phase wird angetrennt und eingeengt. Der Rückstand wird über Kieselgel (Toluol/*n*-Heptan 1:1) geben. Die Endreinigung erfolgt durch Kristallisation aus Acetonitril und n-Heptan.
K 66 SmA 87 N 147 l
Δε 22
Δ n 0,155

Der Synthesebaustein **16** wird anolog Schema 1, WO 2004/048501 A1 hergestellt.

### Beispiel 6

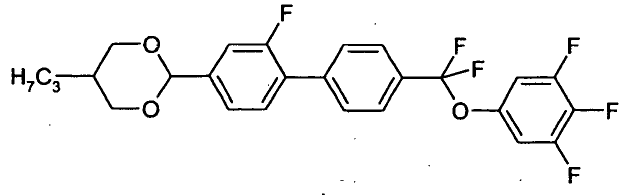

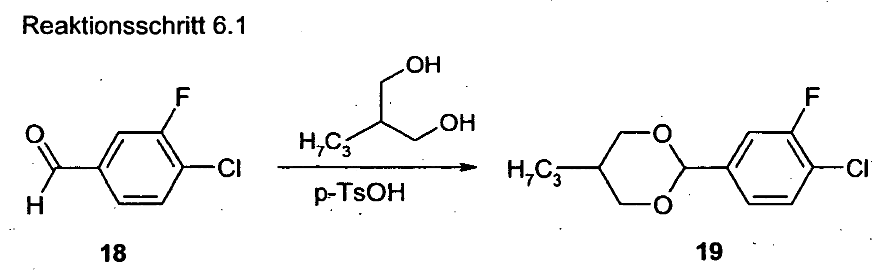

3,2 g (20 mmol) des Aldehyds **18** und 4,7 g (30 mmol) des 1,3-Diols werden in 50 ml Toluol gelöst, mit 0,4 g *p*-Toluolsulfonsäure versetzt und 2 h am Wasserabscheider erhitzt. Die abgekühlte Lösung wird mit ges. Natriumhydrogencarbonat-Lösung gewaschen und eingeengt. Der Rückstand wird über Kieselgel gegeben.

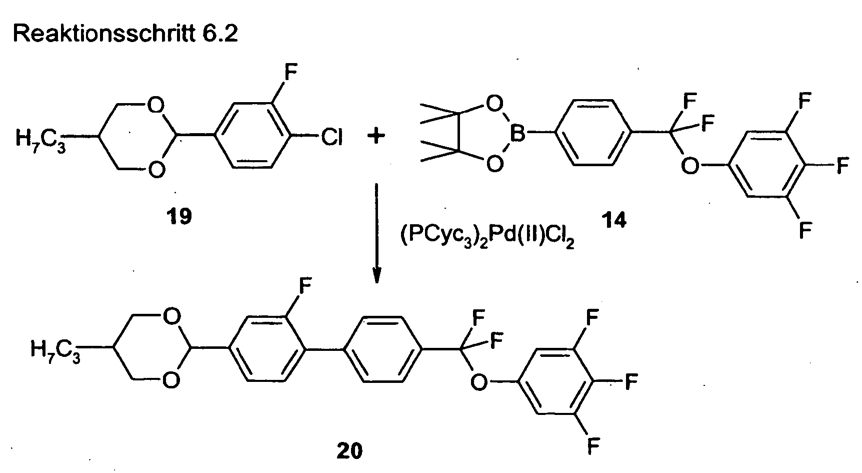

13,9 g (50 mmol) Natriummetaborat-Octahydrat werden in 19 ml Wasser und 18 ml THF vorgelegt und mit 738 mg (1 mmol) (PCyc₃)₂Pd(II)Cl₂ und 0,1 ml (1 mmol) Hydraziniumhydroxid versetzt. Nach 5 min werden 20,8 g (80 mmol) des Boronesters **14,** 12,9 g (50 mmol) des Chlorids **19** und 55 ml THF in den Ansatz gegeben, der 8 h zum Sieden erhitzt wird. Die abgekühlte Reaktionslösung wird mit 200 ml MTB-Ether verdünnt. Die organische Phase wird angetrennt und eingeengt. Der Rückstand wird über Kieselgel (Toluol/*n*-Heptan 1:1) gegeben. Die Endreinigung erfolgt durch Kristallisation aus Acetonitril und n-Heptan.
K 109 SmA (95) N 153 l
Δε 28
Δ n 0,165

### Beispiel 7

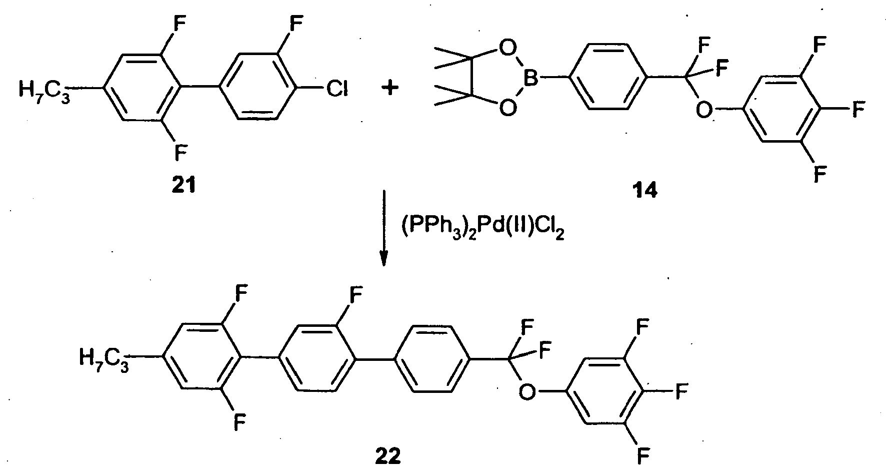

13,9 g (50 mmol) Natriummetaborat-Octahydrat werden in 19 ml Wasser und 18 ml THF vorgelegt und mit 738 mg (1 mmol) (PCyc₃)₂Pd(II)Cl₂ und 0,1 ml (1 mmol) Hydraziniumhydroxid versetzt. Nach 5 min werden 20,8 g (80 mmol) des Boronesters **14**, 14,2 g (50 mmol) des Chlorids **21** und 55 ml THF in den Ansatz gegeben, der 8 h zum Sieden erhitzt wird. Die abgekühlte Reaktionslösung wird mit 200 ml MTB-Ether verdünnt. Die organische Phase wird abgetrennt und eingeengt. Der Rückstand wird über Kieselgel (Toluol/*n*-Heptan 1:1) gegeben. Die Endreinigung erfolgt durch Kristallisation aus Acetonitril und -Heptan.

Der Synthesebaustein **21** wird nach folgendem Schema hergestellt:

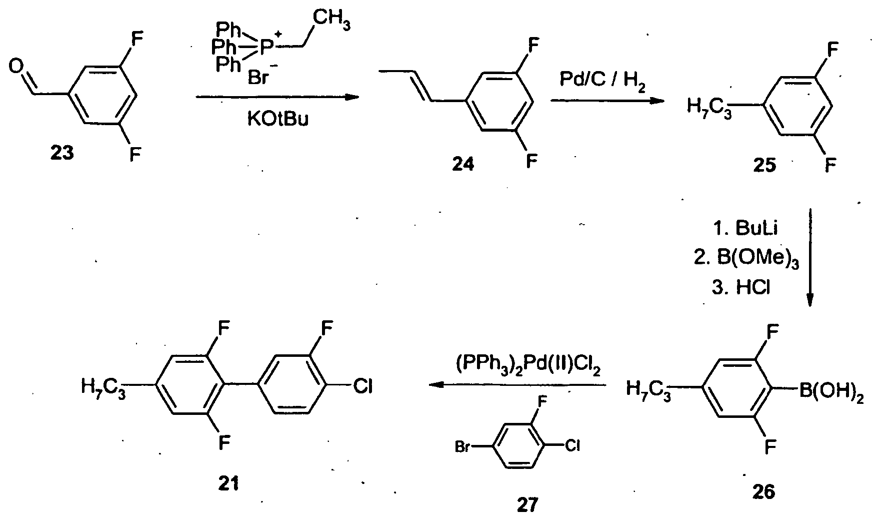

Die Aldehydfunktion in **23** wird in einer Wittig-Reaktion und anschließender Hydrierung in eine Alkylkette - hier eine Propylkette - übergeführt. Über eine Deprotonierung des Aromaten **25** zwischen den Fluoratomen und die Umsetzung mit Trimethylborat erhält man nach saurer Hydrolyse die Boronsäure **26**, die in einer palladiumkatalysierten Boronsäurekopplung mit dem halogenierten Benzol **27** zum Baustein **21** umgesetzt wird.
K 120 N 123 l
Δε 29
Δ n 0,206

Gemäß den beschriebenen Synthesen werden die folgenden weiteren Verbindungen hergestellt (Tabelle 2):

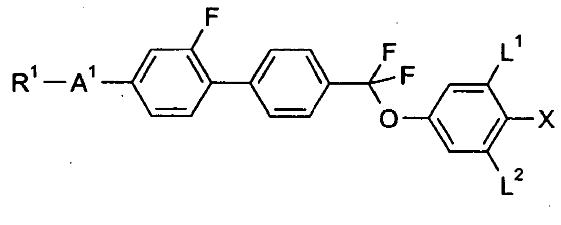

**Tabelle 2**

| R¹ | A¹ | X | L¹ | L² | Werte |
|---|---|---|---|---|---|
| H | 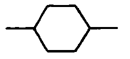 | F | F | H | |
| CH₃ | 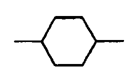 | F | F | H | |
| C₂H₅ | 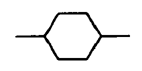 | F | F | H | |
| n-C₃H₇ | 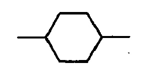 | F | F | H | |
| n-C₄H₉ | 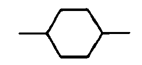 | F | F | H | |
| n-C₅H₁₁ | 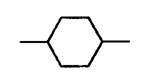 | F | F | H | |
| n-C₆H₁₃ | 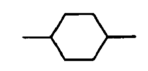 | F | F | H | |
| n-C₇H₁₅ | 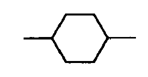 | F | F | H | |
| H | 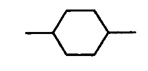 | F | F | F | |
| CH₃ | 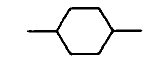 | F | F | F | |
| C₂H₅ | 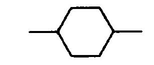 | F | F | F | |
| n-C₃H₇ | 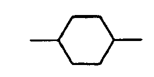 | F | F | F | vgl. Beispiel 2 |
| n-C₄H₉ | 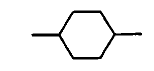 | F | F | F | |
| n-C₅H₁₁ | 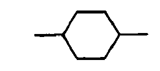 | F | F | F | |
| n-C₆H₁₃ | 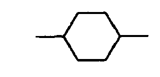 | F | F | F | |
| n-C₇H₁₅ | 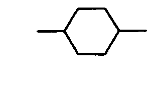 | F | F | F | |
| H | 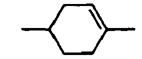 | F | F | H | |
| CH₃ | 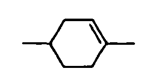 | F | F | H | |
| C₂H₅ | 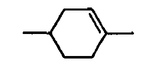 | F | F | H | |
| n-C₃H₇ | 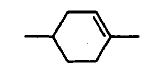 | F | F | H | |
| n-C₄H₉ | 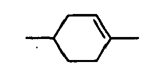 | F | F | H | |
| n-C₅H₁₁ | 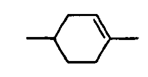 | F | F | H | |
| n-C₆H₁₃ | 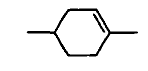 | F | F | H | |
| n-C₇H₁₅ | 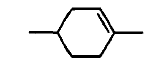 | F | F | H | |
| H | 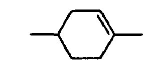 | F | F | F | |
| CH₃ | 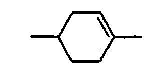 | F | F | F | |
| C₂H₅ | 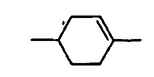 | F | F | F | |
| n-C₃H₇ | 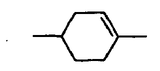 | F | F | F | K 78 SmA 109 N 161 I, |
| | | | | | Δε 22, Δn 0,203 |
| n-C₄H₉ | 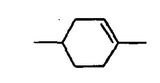 | F | F | F | |
| n-C₅H₁₁ | 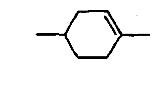 | F | F | F | |
| n-C₆H₁₃ | 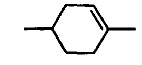 | F | F | F | |
| n-C₇H₁₅ | 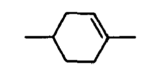 | F | F | F | |
| n-C₃H₇ | 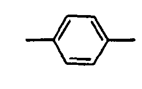 | F | H | H | K 98 SmC (65) SmA' (94) |
| | | | | | SmA 150 N 225 I, |
| | | | | | Δε 12, Δn 0,256 |
| H | 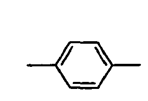 | F | F | H | |
| CH₃ | 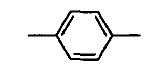 | F | F | H | |
| C₂H₅ | 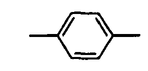 | F | F | H | |
| n-C₃H₇ | 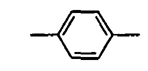 | F | F | H | |
| n-C₄H₉ | 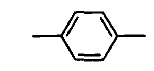 | F | F | H | |
| n-C₅H₁₁ | 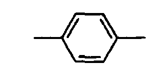 | F | F | H | |
| n-C₆H₁₃ | 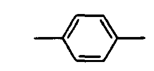 | F | F | H | |
| n-C₇H₁₅ | 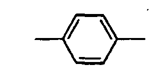 | F | F | H | |
| H | 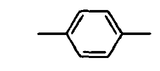 | F | F | F | |
| CH₃ | 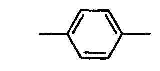 | F | F | F | |
| C₂H₅ | 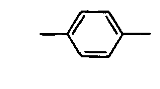 | F | F | F | |
| n-C₃H₇ | 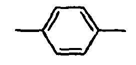 | F | F | F | vgl. Beispiel 3 |
| n-C₄H₉ | 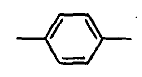 | F | F | F | |
| n-C₅H₁₁ | 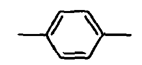 | F | F | F | |
| n-C₆H₁₃ | 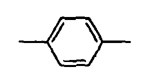 | F | F | F | |
| n-C₇H₅ | 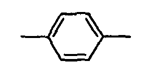 | F | F | F | |
| H | 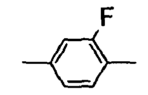 | F | F | H | |
| CH₃ | 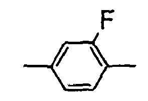 | F | F | H | |
| C₂H₅ | 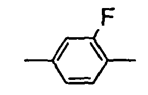 | F | F | H | |
| n-C₃H₇ | 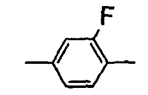 | F | F | H | |
| n-C₄H₉ | 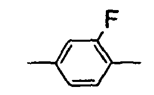 | F | F | H | |
| n-C₅H₁₁ | 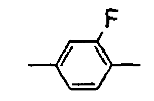 | F | F | H | |
| n-C₆H₁₃ | 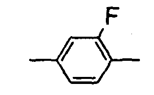 | F | F | H | |
| n-C₇H₁₅ | 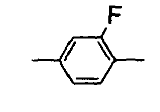 | F | F | H | |
| H | 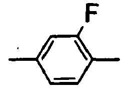 | F | F | F | |
| CH₃ | 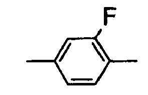 | F | F | F | |
| C₂H₅ | 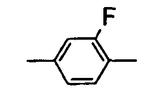 | F | F | F | |
| n-C₃H₇ | 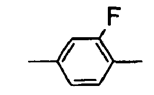 | F | F | F | vgl. Beispiel 4 |
| n-C₄H₉ | 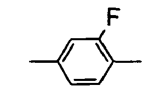 | F | F | F | |
| n-C₅H₁₁ | 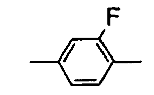 | F | F | F | |
| n-C₆H₁₃ | 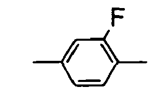 | F | F | F | |
| n-C₇H₁₅ | 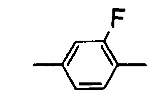 | F | F | F | |
| H | 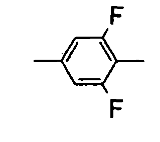 | F | F | H | |
| CH₃ | 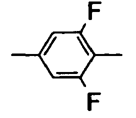 | F | F | H | |
| C₂H₅ | 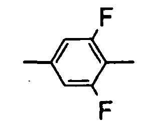 | F | F | H | |
| n-C₃H₇ | 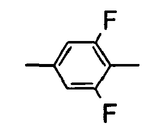 | F | F | H | |
| n-C₄H₉ | 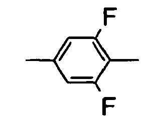 | F | F | H | |
| n-C₅H₁₁ | 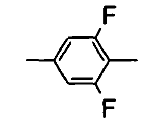 | F | F | H | |
| n-C₆H₁₃ | 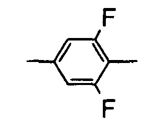 | F | F | H | |
| n-C₇H₁₅ | 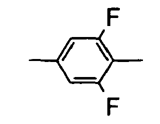 | F | F | H | |
| H | 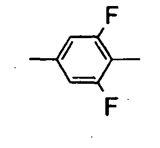 | F | F | F | |
| CH₃ | 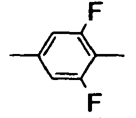 | F | F | F | |
| C₂H₅ | 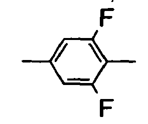 | F | F | F | |
| n-C₃H₇ | 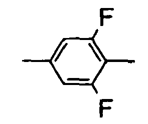 | F | F | F | vgl. Beispiel 7 |
| n-C₄H₉ | 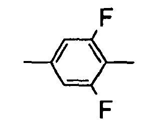 | F | F | F | |
| n-C₅H₁₁ | 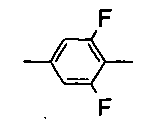 | F | F | F | |
| n-C₆H₁₃ | 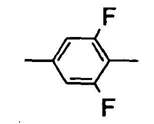 | F | F | F | |
| n-C₇H₁₅ | 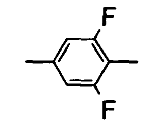 | F | F | F | |
| H | 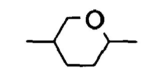 | F | F | H | |
| CH₃ | 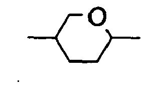 | F | F | H | |
| C₂H₅ | 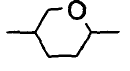 | F | F | H | |
| n-C₃H₇ | 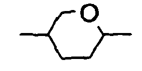 | F | F | H | |
| n-C₄H₉ | 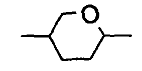 | F | F | H | |
| n-C₅H₁₁ | 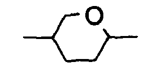 | F | F | H | |
| n-C₆H₁₃ | 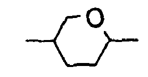 | F | F | H | |
| n-C₇H₁₅ | 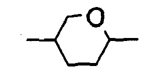 | F | F | H | |
| H | 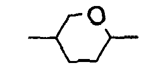 | F | F | F | |
| CH₃ | 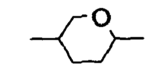 | F | F | F | |
| C₂H₅ | 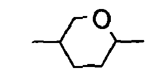 | F | F | F | |
| n-C₃H₇ | 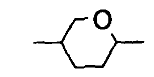 | F | F | F | vgl. Beispiel 5 |
| n-C₄H₉ | 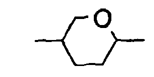 | F | F | F | |
| n-C₅H₁₁ | 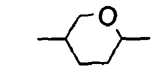 | F | F | F | |
| n-C₆H₁₃ | 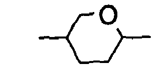 | F | F | F | |
| n-C₇H₁₅ | 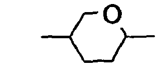 | F | F | F | |
| H | 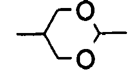 | F | F | H | |
| CH₃ | 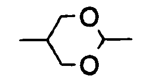 | F | F | H | |
| C₂H₅ | 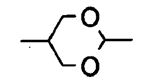 | F | F | H | |
| n-C₃H₇ | 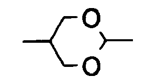 | F | F | H | |
| n-C₄H₉ | 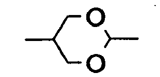 | F | F | H | |
| n-C₅H₁₁ | 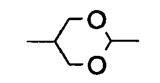 | F | F | H | |
| n-C₆H₁₃ | 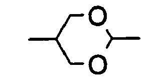 | F | F | H | |
| n-C₇H₁₅ | 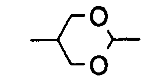 | F | F | H | |
| H | 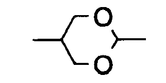 | F | F | F | |
| CH₃ | 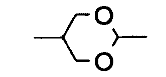 | F | F | F | |
| C₂H₅ | 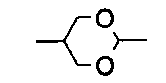 | F | F | F | |
| n-C₃H₇ | 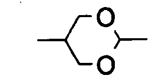 | F | F | F | vgl. Beispiel 6 |
| n-C₄H₉ | 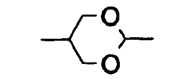 | F | F | F | |
| n-C₅H₁₁ | 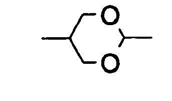 | F | F | F | |
| n-C₆H₁₃ | 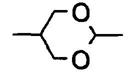 | F | F | F | |
| n-C₇H₁₅ | 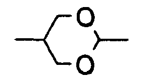 | F | F | F | |
| n-C₃H₇ | 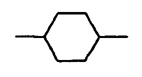 | OCF₃ | F | H | K 42 SmB (30) SmA 131 N |
| | | | | | 190 I, Δε 17, Δn 0,162 |
| n-C₃H₇ | 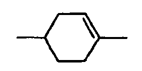 | OCF₃ | F | H | K 31 Sm (18) SmA 177 N |
| | | | | | 193 I, Δε 18, Δn 0,197 |
| n-C₃H₇ | 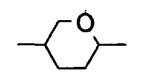 | OCF₃ | F | H | K 65 SmA 162 N 179 I, |
| | | | | | Δε 19, Δn 0,193 |
| n-C₃H₇ | 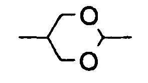 | OCF₃ | F | H | K 58 SmA 170 N 181 I, |
| | | | | | Δε 24, An 0,157 |
| n-C₃H₇ | 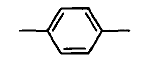 | OCF₃ | F | H | K 68 SmB 80 SmA 208 N |
| | | | | | 213 I, Δε 17, An 0,233 |

Weitere Kombinationen der Ausführungsformen und Varianten der Erfindung gemäß der Beschreibung ergeben sich auch aus den folgenden Ansprüchen.

## Patentansprüche

1. Verbindungen der Formel I, 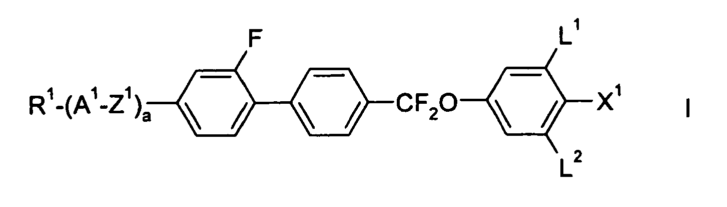 worin
R¹ H, F, C), Br, einen halogenierten oder unsubstituierten Alkylrest mit 1 bis 15 C-Atomen, wobei in diesen Resten auch eine oder mehrere CH₂-Gruppen jeweils unabhängig voneinander durch -C≡C-, -(CO)O-, -O(CO)-, -(CO)- oder -O- so ersetzt sein können, dass O-Atome nicht direkt miteinander verknüpft sind,
A¹
a) trans-1,4-Cyclohexylen oder Cyclohexenylen, worin auch eine oder mehrere nicht benachbarte CH₂-Gruppen durch -O- und/oder -S- ersetzt sein können und worin H durch F substituiert sein kann,
b) 1,4-Phenylen, worin eine oder zwei CH-Gruppen durch N ersetzt sein können und worin auch ein oder mehrere H- Atome gegen Br, Cl, F, CN, Methyl, Methoxy oder eine ein- oder mehrfach fluorierte Methyl- oder Methoxygruppe ersetzt sein können,
Z¹ eine Einfachbindung, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- oder -C≡C-, wobei asymmetrische Brücken nach beiden Seiten orientiert sein können,
L¹ und L² unabhängig voneinander H oder F,
X¹ F, Cl, CN, SF₅, einen halogenierten Alkylrest, halogenierten Alkoxyrest, halogenierten Alkenylrest oder halogenierten Alkenyloxyrest mit jeweils bis zu 7 C-Atomen, und
a 0, 1 oder 2,
bedeuten.

2. Verbindungen nach Anspruch 1 der Formel IA 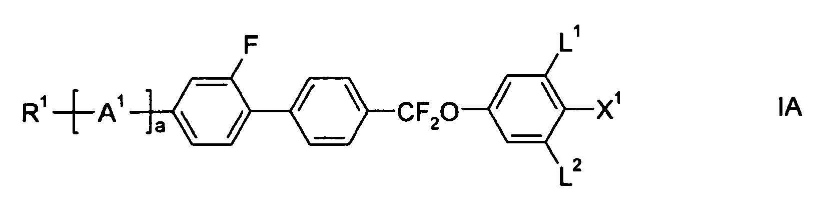 worin
R¹, A¹, L¹, L², X¹ und a die in Anspruch 1 für Formel I angegebenen Bedeutungen haben.

3. Verbindungen nach Anspruch 1 oder 2, **dadurch gekennzeichnet, dass**
R¹ Alkyl oder Alkoxy mit bis zu 8 Kohlenstoffatomen
bedeutet.

4. Verbindungen nach einem oder mehreren der Ansprüche 1 bis 3, **dadurch gekennzeichnet, dass** L¹ Fluor und L² Fluor oder Wasserstoff bedeuten.

5. Verbindungen nach einem oder mehreren der Ansprüche 1 bis 4 der Formeln I1 bis I8, 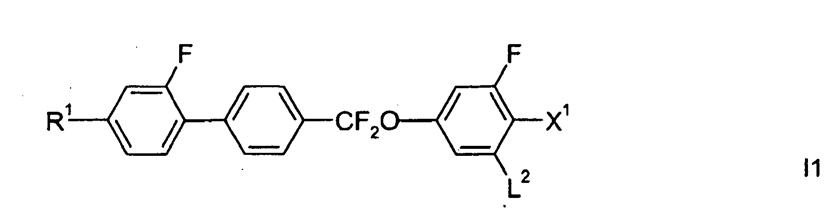 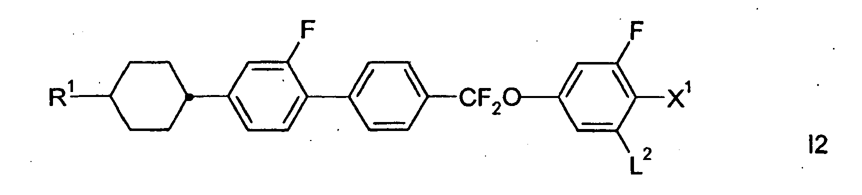 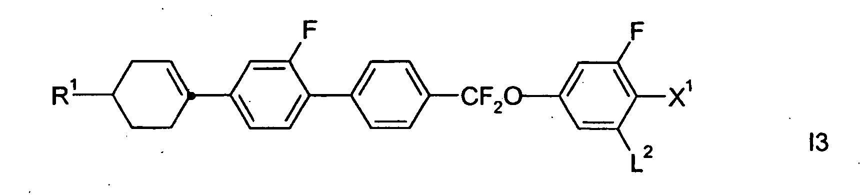 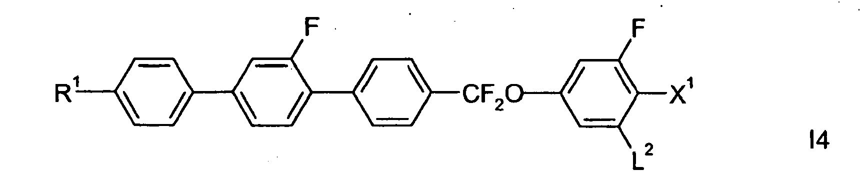 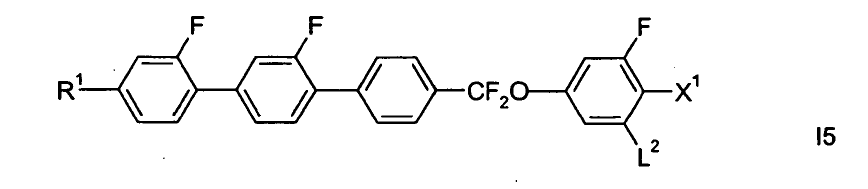 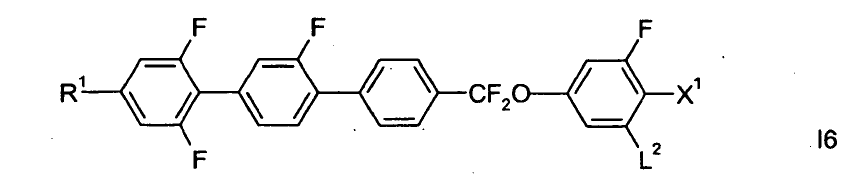 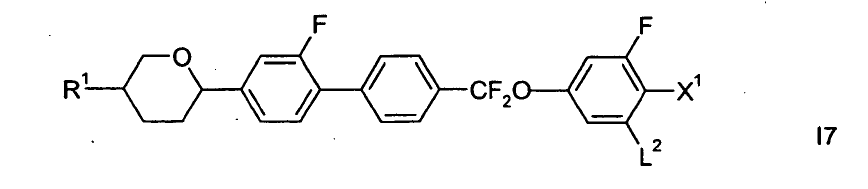 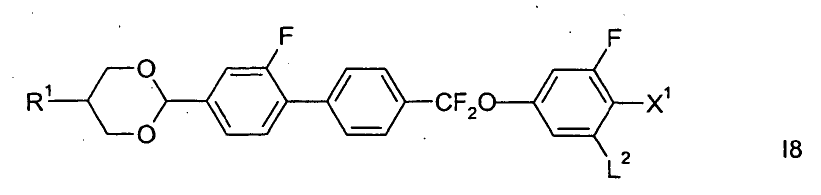 worin R¹, L² und X¹ die in einem oder mehreren der Ansprüche 1 bis 4 angegebenen Bedeutungen haben.

6. Verbindungen nach einem oder mehreren der Ansprüche 1 bis 5, **dadurch gekennzeichnet, dass** L¹ und L² Fluor bedeuten.

7. Verfahren zur Herstellung von Verbindungen der Formel I nach einem oder mehreren der Ansprüche 1 bis 6 umfassend einen Verfahrensschritt, in dem eine Boronsäure der Formel IIA oder ein offenkettiger oder cyclischer Boronsäureester der Formel IIB 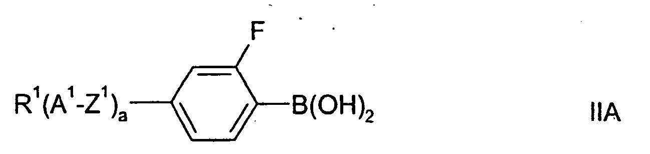 oder 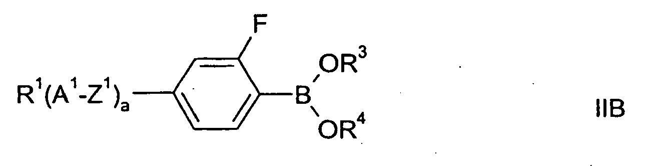 worin R¹, A¹, Z¹ und a wie in Anspruch 1 definiert sind, und
R³, R⁴ ein Alkyl mit 1-12 C-Atomen oder R³+R⁴ zusammen auch ein Alkylen, oder 1,2-Phenylen bedeuten, wobei Phenylen, R³, R⁴ und R³+R⁴ auch substituiert sein können und wobei p 0 oder 1 ist,
mit einer Verbindung der Formel III 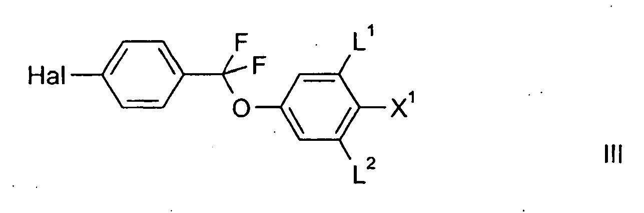 worin L¹, L² und X¹ wie in Anspruch 1 definiert sind und
Hal O(SO₂)CF₃, Cl, Br oder I bedeutet,
in Gegenwart eines Übergangsmetallkatalysators zur Reaktion gebracht wird.

8. Verfahren zur Herstellung von Verbindungen der Formel I nach einem oder mehreren der Ansprüche 1 bis 6 umfassend einen Verfahrensschritt, in dem eine Verbindung mit einer Abgangsgruppe Hal' der Formel IV 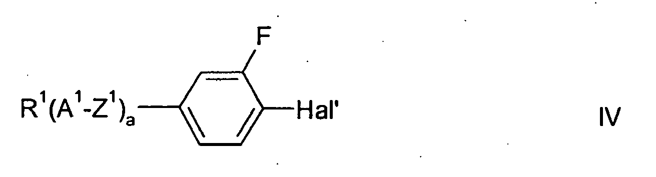 worin R¹, A¹, Z¹ und a wie in Anspruch 1 definiert sind, und
Hal' -O(SO₂)CF₃, Cl, Br oder I bedeutet,
mit einer Boronsäure oder einem offenkettigen oder cyclischen Boronsäureester der Formel V 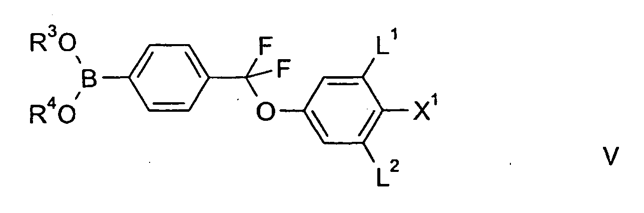 worin L¹, L² und X¹ wie in Anspruch 1 definiert sind und
R³, R⁴ H, ein Alkyl mit 1-12 C-Atomen oder R³+R⁴ zusammen auch ein Alkylen oder 1,2-Phenylen bedeuten,
wobei Phenylen, R³, R⁴ und R³+R⁴ auch substituiert sein können und wobei p 0 oder 1 ist,
in Gegenwart eines Übergangsmetallkatalysators zur Reaktion gebracht wird.

9. Verwendung einer oder mehrerer Verbindungen der Formel I nach einem oder mehreren der Ansprüche 1 bis 6 als Komponenten in einem flüssigkristallinen Medium.

10. Flüssigkristallines Medium enthaltend mindestens zwei mesogene Verbindungen, **dadurch gekennzeichnet, dass** es mindestens eine Verbindung der Formel I nach einem oder mehreren der Ansprüche 1 bis 6 enthält.

11. Verwendung des flüssigkristallinen Mediums nach Anspruch 10 für elektrooptische Zwecke.

12. Elektrooptische Flüssigkristallanzeige enthaltend ein flüssigkristallines Medium nach Anspruch 10.

## Claims

1. Compounds of the formula I 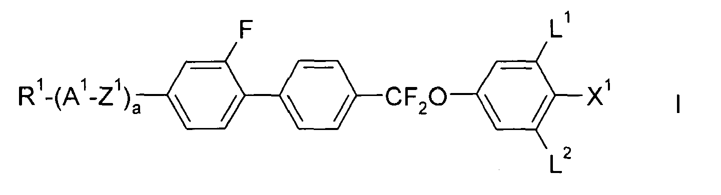 in which
R¹ denotes H, F, Cl, Br, a halogenated or unsubstituted alkyl radical having 1 to 15 C atoms, where, in addition, one or more CH₂ groups in these radicals may each, independ- ently of one another, be replaced by -C≡C-, -(CO)O-, -O(CO)-, -(CO)- or -O- in such a way that O atoms are not linked directly to one another,
A¹ denotes
a) trans-1,4-cyclohexylene or cyclohexenylene, in which, in addition, one or more non-adjacent CH₂ groups may be replaced by -O- and/or -S- and in which H may be substi- tuted by F,
b) 1,4-phenylene, in which one or two CH groups may be replaced by N and in which, in addition, one or more H atoms may be replaced by Br, Cl, F, CN, methyl, methoxy or a mono- or polyfluorinated methyl or methoxy group,
Z¹ denotes a single bond, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- or -C≡C-, where asymmetri- cal bridges may be oriented to both sides,
L¹ and L², independently of one another, denote H or F,
X¹ denotes F, Cl, CN, SF₅, a halogenated alkyl radical, halo- genated alkoxy radical, halogenated alkenyl radical or halogenated alkenyloxy radical, each having up to 7 C atoms, and
a denotes 0, 1 or 2.

2. Compounds according to Claim 1 of the formula IA 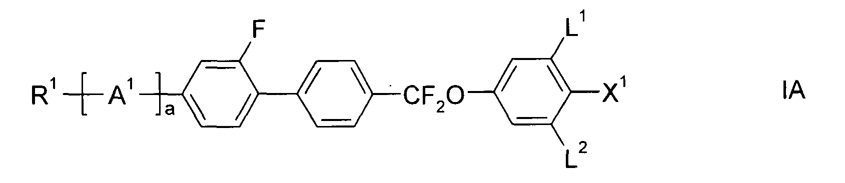 in which
R¹, A¹, L¹, L², X¹ and a have the meanings indicated for formula I in Claim 1.

3. Compounds according to Claim 1 or 2, **characterised in that**
R¹ denotes alkyl or alkoxy having up to 8 carbon atoms.

4. Compounds according to one or more of Claims 1 to 3, **characterised in that** L¹ denotes fluorine and L² denotes fluorine or hydrogen.

5. Compounds according to one or more of Claims 1 to 4 of the formulae I1 to I8 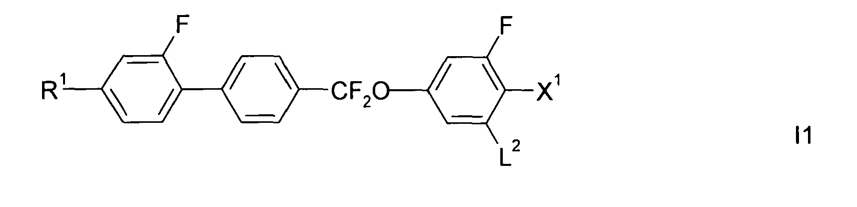 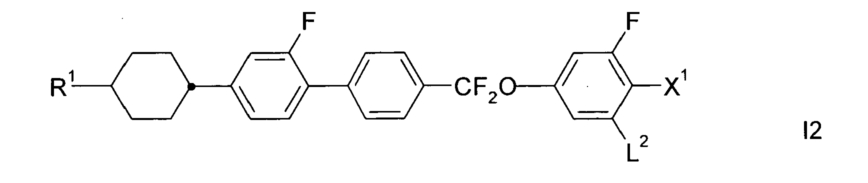 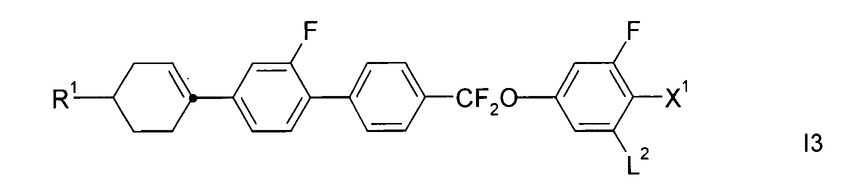 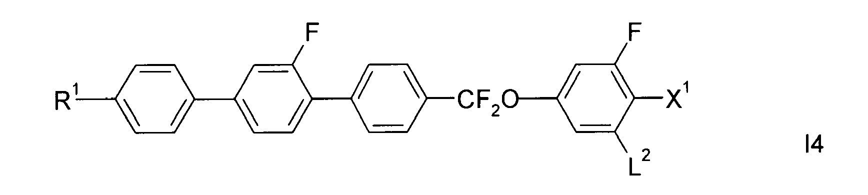 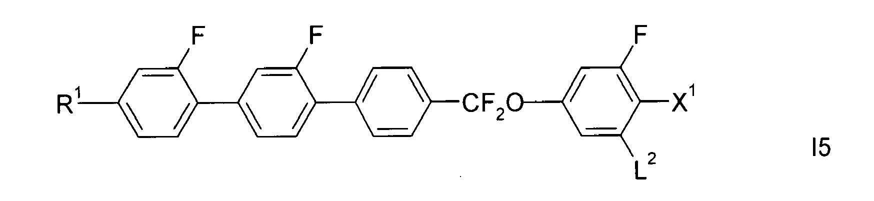 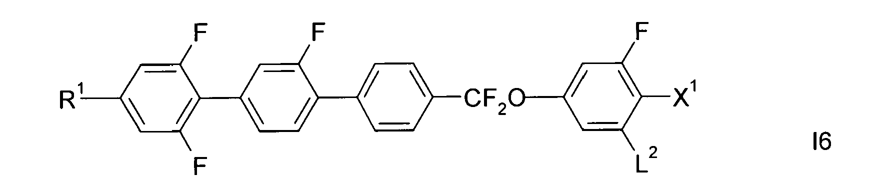 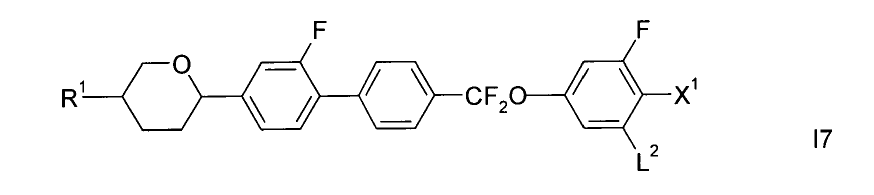 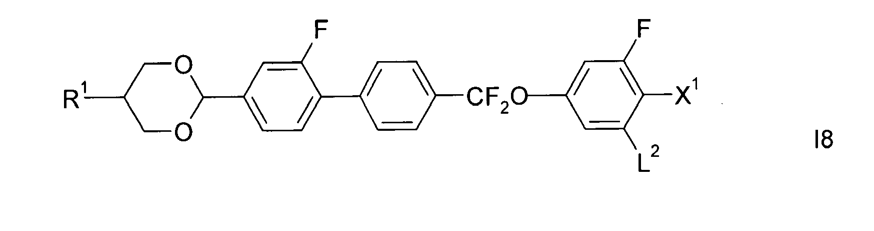 in which R¹, L² and X¹ have the meanings indicated in one or more of Claims 1 to 4.

6. Compounds according to one or more of Claims 1 to 5, **characterised in that** L¹ and L² denote fluorine.

7. Process for the preparation of compounds of the formula I according to one or more of Claims 1 to 6 comprising a process step wherein a boronic acid of the formula IIA or an open-chain or cyclic boronic acid ester of the formula IIB 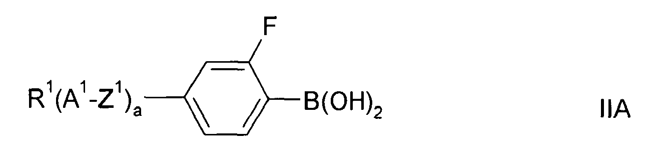 or 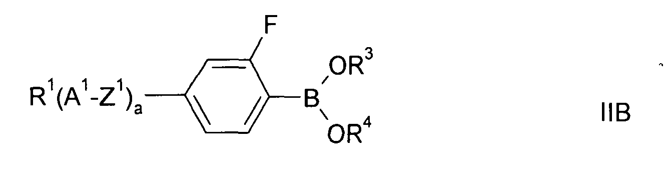 in which R¹, A¹, Z¹ and a are as defined in Claim 1, and
R³, R⁴ denote an alkyl having 1-12 C atoms or R³+R⁴ together also denote an alkylene or 1,2-phenylene,
where phenylene, R³, R⁴ and R³+R⁴ may also be substituted and where p is 0 or 1,
is reacted with a compound of the formula III 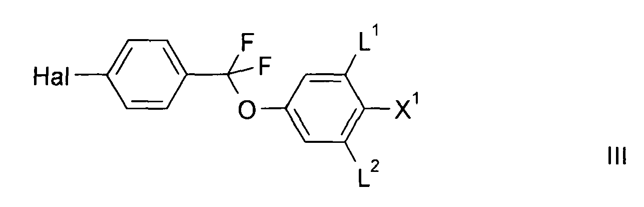 in which L¹, L² and X¹ are as defined in Claim 1, and
Hal denotes O(SO₂)CF₃, Cl, Br or I,
in the presence of a transition-metal catalyst.

8. Process for the preparation of compounds of the formula I according to one or more of Claims 1 to 6 comprising a process step wherein a compound containing a leaving group Hal', of the formula IV 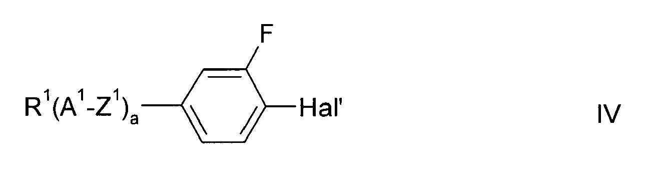 in which R¹, A¹, Z¹ and a are as defined in Claim 1, and
Hal' denotes -O(SO₂)CF₃, Cl, Br or I,
is reacted with a boronic acid or an open-chain or cyclic boronic acid ester of the formula V 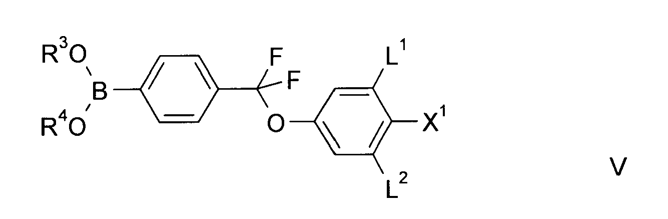 in which L¹, L² and X¹ are as defined in Claim 1, and
R³, R⁴ denote H, an alkyl having 1-12 C atoms or R³+R⁴ together also denote an alkylene or 1,2-phenylene, where phenylene, R³, R⁴ and R³+R⁴ may also be substituted and where p is 0 or 1,
in the presence of a transition-metal catalyst.

9. Use of one or more compounds of the formula I according to one or more of Claims 1 to 6 as components in a liquid-crystalline medium.

10. Liquid-crystalline medium comprising at least two mesogenic compounds, **characterised in that** it comprises at least one compound of the formula I according to one or more of Claims 1 to 6.

11. Use of the liquid-crystalline medium according to Claim 10 for electro-optical purposes.

12. Electro-optical liquid-crystal display containing a liquid-crystalline medium according to Claim 10.

## Revendications

1. Composés de la formule I 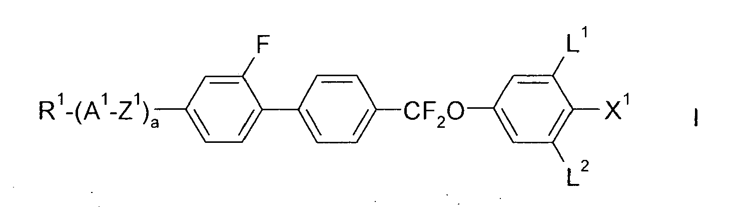 dans laquelle
R¹ représente H, F, Cl, Br, un radical alkyle halogéné ou non substitué ayant 1 à 15 atomes de C, où, en addition, un ou plusieurs groupes CH₂ dans ces radicaux peuvent chacun, indépendamment l'un de l'autre, être remplacés par -C≡C-, -(CO)O-, -O(CO)-, -(CO)- ou -O- de telle sorte que des atomes de O ne soient pas liés directement l'un à l'autre,
A¹ représente
a) trans-1,4-cyclohexylène ou cyclohexénylène, dans lequel, en addition, un ou plusieurs groupes CH₂ non adja- cents peuvent être remplacés par -O- et/ou -S- et dans lequel H peut être substitué par F,
b) 1,4-phénylène, dans lequel un ou deux groupes CH peuvent être remplacés par N et dans lequel, en addition, un ou plusieurs atomes de H peuvent être remplacés par Br, Cl, F, CN, méthyle, méthoxy ou un groupe méthyle ou méthoxy mono- ou polyfluoré,
Z¹ représente une liaison simple, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- ou -C≡C-, où des pontages asymétriques peuvent être orientés sur les deux côtés,
L¹ et L², indépendamment l'un de l'autre, représentent H ou F,
X¹ représente F, Cl, CN, SF₅, un radical alkyle halogéné, radical alcoxy halogéné, radical alkényle halogéné ou radi- cal alkényloxy halogéné, chacun ayant jusqu'à 7 atomes de C, et
a représente 0,1 ou 2.

2. Composés selon la revendication 1 de la formule IA 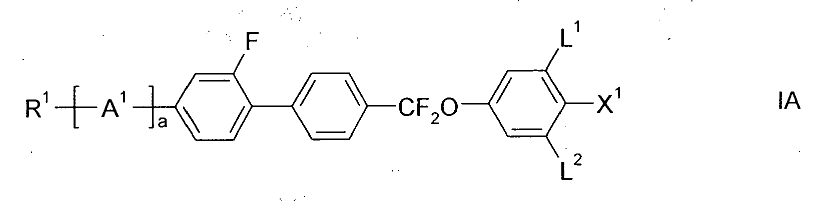 dans laquelle
R¹, A¹, L¹, L², X¹ et a ont les significations indiquées pour la formule I dans la revendication 1.

3. Composés selon la revendication 1 ou 2, **caractérisés en ce que**
R¹ représente alkyle ou alcoxy ayant jusqu'à 8 atomes de car- bone.

4. Composés selon une ou plusieurs des revendications 1 à 3, **caractérisés en ce que** L¹ représente fluor et L² représente fluor ou hydrogène.

5. Composés selon une ou plusieurs des revendications 1 à 4 des formules I1 à I8 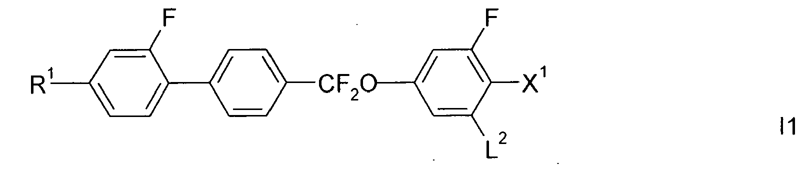 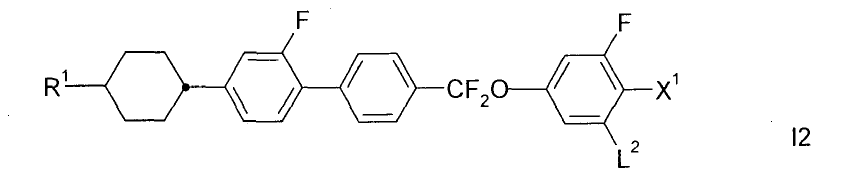 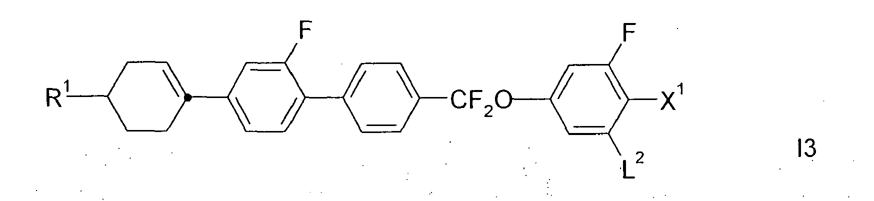 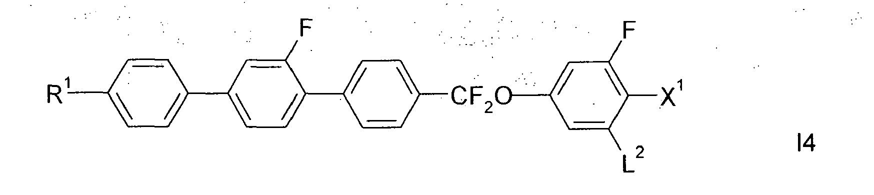 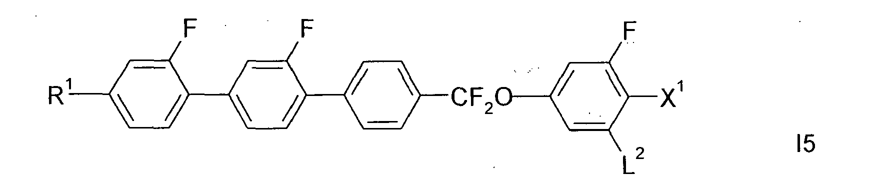 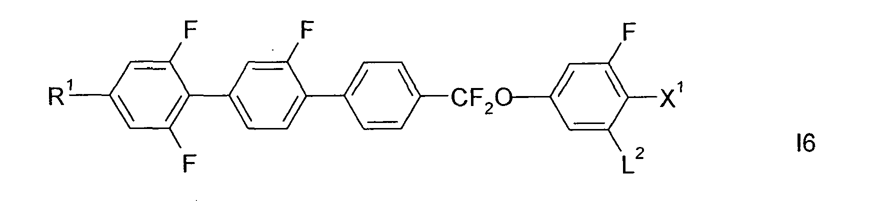 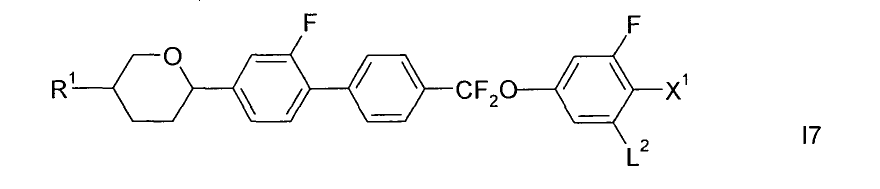 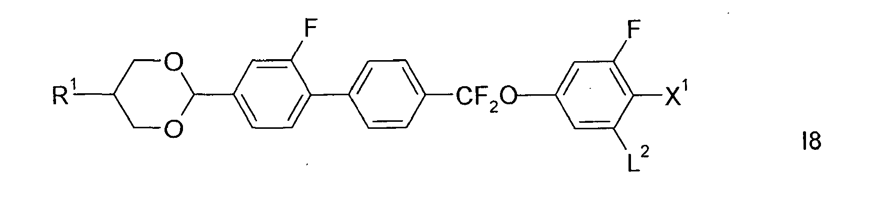 dans lesquelles R¹, L² et X¹ ont les significations indiquées dans une ou plusieurs des revendications 1 à 4.

6. Composés selon une ou plusieurs des revendications 1 à 5, **caractérisés en ce que** L¹ et L² représentent fluor.

7. Procédé pour la préparation de composés de la formule I selon une ou plusieurs des revendications 1 à 6 comprenant une étape de procédé selon laquelle un acide boronique de la formule IIA ou un ester d'acide boronique en chaîne ouverte ou cyclique de la formule IIB 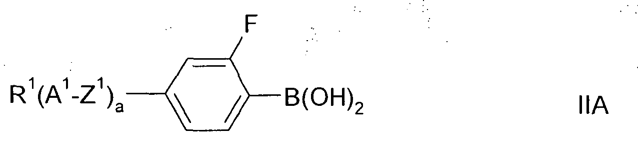 ou 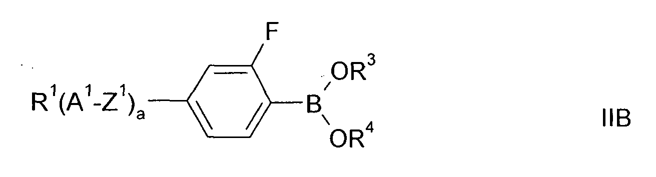 dans laquelle R¹, A¹, Z¹ et a sont comme définis dans la revendication 1, et
R³, R⁴ représentent un alkyle ayant 1-12 atomes de C ou R³+R⁴ ensemble représentent également un alkylène ou 1,2- phénylène,
où phénylène, R³, R⁴ et R³+R⁴ peuvent également être subs- titués et où p est 0 ou 1,
est amené à réagir avec un composé de la formule III 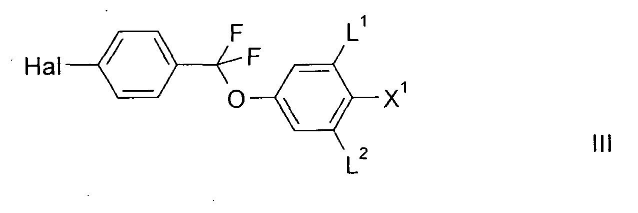 dans laquelle L¹, L² et X¹ sont comme définis dans la revendication 1, et
Hal représente O(SO₂)CF₃, Cl, Br ou I,
en présence d'un catalyseur à métal de transition.

8. Procédé pour la préparation de composés de la formule I selon une ou plusieurs des revendications 1 à 6 comprenant une étape de procédé selon laquelle un composé contenant un groupe quittant Hal', de la formule IV 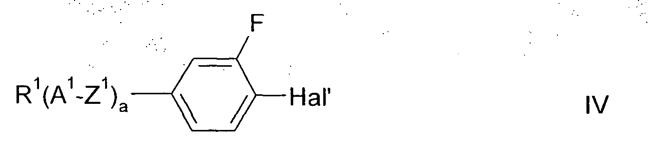 dans laquelle R¹, A¹, Z¹ et a sont comme définis dans la revendication 1, et
Hal' représenté -O(SO₂)CF₃, Cl, Br ou I,
est amené à réagir avec un acide boronique ou un ester d'acide boronique en chaîne ouverte ou cyclique de la formule V 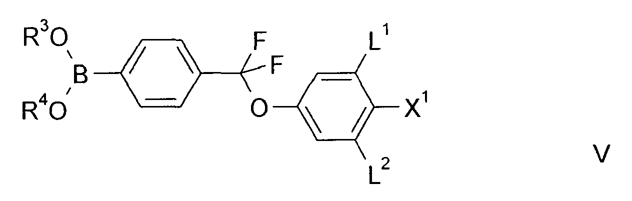 dans laquelle L¹, L² et X¹ sont comme définis dans la revendication 1, et
R³, R⁴ représentent H, un alkyle ayant 1-12 atomes de C ou R³+R⁴ ensemble représentent également un alkylène ou 1,2- phénylène,
où phénylène, R³, R⁴ et R³+R⁴ peuvent également être subs- titués et où p est 0 ou 1,
en présence d'un catalyseur à métal de transition.

9. Utilisation d'un ou plusieurs composés de la formule I selon une ou plusieurs des revendications 1 à 6 comme composants dans un milieu cristallin liquide.

10. Milieu cristallin liquide comprenant au moins deux composés mésogènes, **caractérisé en ce qu'**il comprend au moins un composé de la formule I selon une ou plusieurs des revendications 1 à 6.

11. Utilisation du milieu cristallin liquide selon la revendication 10 à des fins électro-optiques.

12. Affichage à cristaux liquides électro-optique contenant un milieu cristallin liquide selon la revendication 10.
